# EUROPEAN PATENT APPLICATION

(11) **EP 4 656 232 A1**
(43) Date of publication of application: **03.12.2025**
(21) Application number: 24818379.0
(22) Date of filing: 17.04.2024
(51) Int. Cl.: A61M 60/139, A61M 60/216, A61M 60/416, A61M 60/857, A61M 60/859, A61M 60/802, A61M 60/90, A61M 60/13, A61M 60/174, A61M 60/221, A61M 60/33, A61M 60/81, A61M 60/829

(54) **BLOOD PUMP**

(30) Priority: 06.06.2023 CN 202310661447; 06.06.2023 CN 202310664727; 06.06.2023 CN 202310662718; 06.06.2023 CN 202310667240
(71) Applicant: Shenzhen Core Medical Technology Co., Ltd., Shenzhen, Guangdong 518000 (CN)
(72) Inventor: YANG, Yuzhuo, Shenzhen, Guangdong 518000 (CN); XIAO, Zhenzhong, Shenzhen, Guangdong 518000 (CN); YU, Shunzhou, Shenzhen, Guangdong 518000 (CN)
(74) Representative: Murgitroyd & Company
(86) International application number: PCT/CN2024/088190
(87) International publication number: WO 2024/250843

(57) **Abstract**

A blood pump (10). The blood pump (10) comprises a primary pumping device (100), a first conduit (200), a secondary pumping device (300) and a second conduit (400), which are sequentially connected, wherein the primary pumping device (100) is provided with a first inlet (101) and a first outlet (102); and the secondary pumping device (300) is provided with a second inlet (301) and a second outlet (302), the second inlet (301) being arranged on an outer peripheral wall of the secondary pumping device (300).

## Description

This application claims priority to Chinese Patent Application No. CN202310661447.2, entitled "Blood Pump", Chinese Patent Application No. CN202310664727.9, entitled "Blood Pump", Chinese Patent Application No. CN202310662718.6, entitled "Blood Pump", and Chinese Patent Application No. CN202310667240.6, entitled "Blood Pump", filed with China National Intellectual Property Administration on June 6, 2023, disclosures of which are incorporated herein by reference in their entities.

### TECHNICAL FIELD

The present application relates to the field of medical instrument technologies, and in particular, to a blood pump.

### BACKGROUND

As a cardiac assist device, a blood pump is usually employed to partially or completely replace the work of the heart, so as to assist blood circulation of a patient. However, the conventional blood pump often has the defects of insufficient power for driving blood to flow, or the like.

### SUMMARY

Accordingly, the present application provides a blood pump, which is intended to solve the problem of an insufficient driving force of the conventional blood pump.

In a first aspect, embodiments of the present application provide a blood pump, including: a primary pumping device, a first catheter, a secondary pumping device and a second catheter which are connected in sequence; wherein the primary pumping device is provided with a first inlet and a first outlet; the secondary pumping device is provided with a second inlet and a second outlet, and the second inlet is arranged in an outer peripheral wall of the secondary pumping device.

Details of one or more embodiments of the present invention are set forth in the accompanying drawings and the description below. Other features, objects, and advantages of the present invention will be apparent from the description, the accompanying drawings, and the claims.

### BRIEF DESCRIPTION OF THE DRAWINGS

In order to illustrate the technical solutions in the embodiments of the present application more clearly, the drawings required for describing the embodiments or the prior art will be introduced briefly. Apparently, the following described drawings are merely for some embodiments of the present application, and other drawings can be derived from these drawings by those of ordinary skill in the art without any creative effort.
FIG. 1 is a schematic structural view of a blood pump according to an embodiment of the present application.
FIG. 2 is a sectional view of the blood pump in FIG. 1 extending through the aorta.
FIG. 3 is a schematic view of a secondary pumping device connected to a first catheter and a second catheter in FIG. 1.
FIG. 4 is an exploded view showing an assembly structure of the secondary pumping device, the first catheter and the second catheter in FIG. 3.
FIG. 5 is a schematic view of a partial structure of the secondary pumping device in FIG. 3.
FIG. 6 is an exploded view of the secondary pumping device in FIG. 5.
FIG. 7 is a sectional view of the secondary pumping device in FIG. 3 taken along a line A-A.
FIG. 8 is a schematic enlarged view at portion P₁ in FIG. 7.
FIG. 9 is a schematic enlarged view at portion P₂ in FIG. 7.
FIG. 10 is a schematic enlarged view at portion P₃ in FIG. 7.
FIG. 11 is a schematic structural view of a proximal part of the secondary pumping device in FIG. 3.
FIG. 12 is a schematic enlarged view at portion P₄ in FIG. 11.
FIG. 13 is a schematic view showing a liquid inlet groove in FIG. 12 formed by a fixed tube cooperating with a rotating shaft and a proximal bearing.
FIG. 14 is a sectional view of the secondary pumping device in FIG. 3 taken along a line B-B.
FIG. 15 is a schematic view of assembling of the rotating shaft, the fixed tube and a secondary impeller in FIG. 7.
FIG. 16 is an axial sectional view of the assembled rotating shaft, fixed tube and secondary impeller in FIG. 15.
FIG. 17 is a partial enlarged view of the assembled rotating shaft, fixed tube and secondary impeller in FIG. 16.
FIG. 18 is a schematic view of assembling of a rotating shaft and a fixed tube of the secondary pumping device in an embodiment of the present application.
FIG. 19 is a schematic view of the rotating shaft and the fixed tube in FIG. 18 that are separated from each other.
FIG. 20 is a schematic structural view of the fixed tube in FIG. 19 in another embodiment.
FIG. 21 is a transverse sectional view of the rotating shaft and the fixed tube in FIG. 18 that are in one of cooperation manners.
FIG. 22 is a schematic structural view of the assembled fixing pin and the proximal bearing in FIG. 7.
FIG. 23 is a schematic view of an inner structure of the fixing pin in FIG. 22.
FIG. 24 is a schematic view of another example of a secondary pumping device in yet another embodiment of the present application.
FIG. 25 is a schematic view of a secondary cannula in FIG. 24 through which blood flows.
FIG. 26 is a schematic structural view of the secondary cannula of the secondary pumping device in FIG. 24.
FIG. 27 is a front view of the secondary cannula in FIG. 26.
FIG. 28 is a schematic view of a blood flow direction in a conventional straight cannula in the aorta.
FIG. 29 is a schematic structural view of a primary pumping device in an embodiment of the present application.

### DETAILED DESCRIPTION

In order to make the above objects, features and advantages of the present application more apparent, the embodiments of the present application are described below in detail with reference to the accompanying drawings. In the following description, numerous specific details are set forth, so as to provide a thorough understanding of the present application. However, the present application may be implemented in many ways different from those described herein, those skilled in the art may make similar improvements without departing from the essence of the present application, and therefore, the present application is not limited to the embodiments disclosed below.

In descriptions of the present application, it should be understood that, directions or positional relationships indicated by terms "center", "longitudinal", "transverse", "length", "width", "thickness", "upper", "lower", "front", "rear", "left", "right", "vertical", "horizontal", "top", "bottom", "inner", "outer", "clockwise", "anticlockwise", "axial", "radial", "circumferential" etc. are based on orientations or positional relationships shown in the accompanying drawings, and they are used only for the convenience of describing the present application and for description simplicity, but do not indicate or imply that an indicated device or element must have a specific orientation or be constructed and operated in a specific orientation. Therefore, it cannot be understood as a limitation on the present application.

In addition, if the terms such as "first" and "second" appear, the terms are used herein for purposes of description and are not construed as indicating or implying relative importance or significance or to implicitly indicating the number of indicated technical features. Thus, the feature defined with "first" and "second" may include at least one of the features explicitly or implicitly. In the description of the present application, "a plurality of" means at least two, such as two, three, or the like, unless otherwise explicitly specified.

In the present application, unless explicitly specified or limited otherwise, if the term "mounting", "connection", "coupling", or "fixation" or the like appear, the terms should be understood broadly. It may be, for example, a fixed connection, a detachable connection, or an integral formation; or may also be a mechanical or electrical connection; or may also be a direct connection or an indirect connection via an intermediate medium; or may also be a communication inside two elements or an interaction relationship between two elements, unless otherwise explicitly specified. The specific meanings of the above terms can be understood by those skilled in the art according to specific situations.

In the present application, unless explicitly specified or limited otherwise, if a first feature is described as being "on" or "below" a second feature, or the like, it may mean that the first and second features are in direct contact, or that the first and second features are in indirect contact via an intermediate medium. Furthermore, when a first feature is "above", "on" or "on top of" a second feature, it means that the first feature is directly above or obliquely above the second feature, or it simply means that the first feature is higher in level than the second feature. When a first feature is "below", "under" or "underneath" a second feature, it means that the first feature is directly below or obliquely below the second feature, or it simply means that the first feature is lower in level than the second feature.

It should be noted that when an element is referred to as being "fixed on" or "disposed at" another element, the element may be directly located on the other element or an intermediate element may exist. If one element is considered to be "connected" to another element, it may be directly connected to the other element or an intermediate element may exist at the same time. If present, the terms "vertical", "horizontal", "upper", "lower", "left", "right" and similar expressions used in this application are for illustrative purposes only and do not represent the unique implementations.

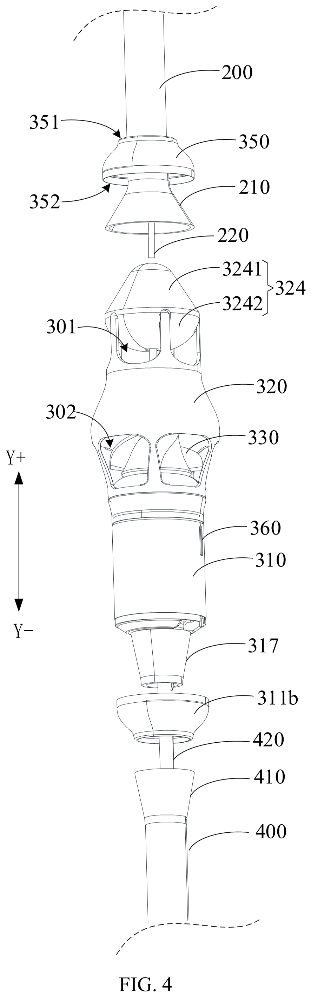

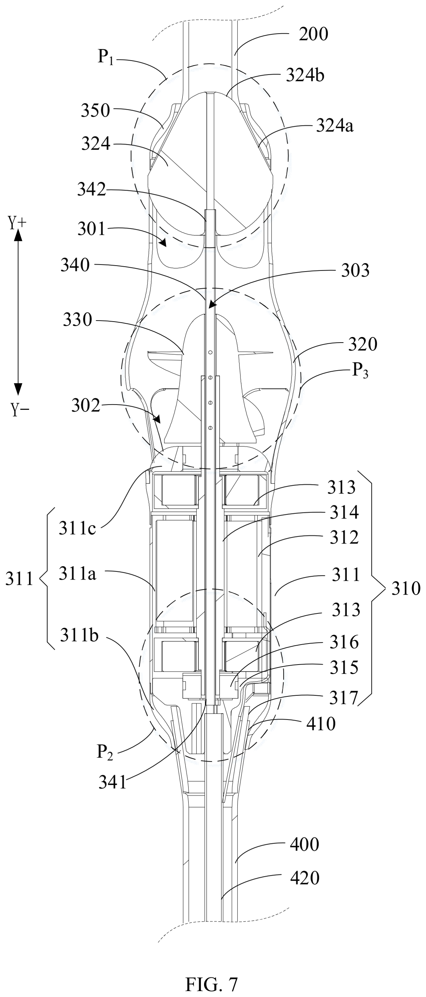

The present application provides an embodiment of a blood pump. The blood pump can be employed to assist blood flow in the right ventricle and can also be employed to assist blood flow in the left ventricle, which is not limited herein. In order to avoid repeated descriptions, the following description is given with the blood pump employed to assist the blood flow in the right ventricle as an example. For ease of description, herein, a "proximal end" is defined as the end of the interventional medical device closer to an operator, and a "distal end" is defined as the end of the interventional medical device farther from the operator, but which is not limited. For example, as shown in FIG. 4 and FIG. 7, Y+ represents the direction from the "proximal end" to the "distal end", and Y- represents the direction from the "distal end" to the "proximal end".

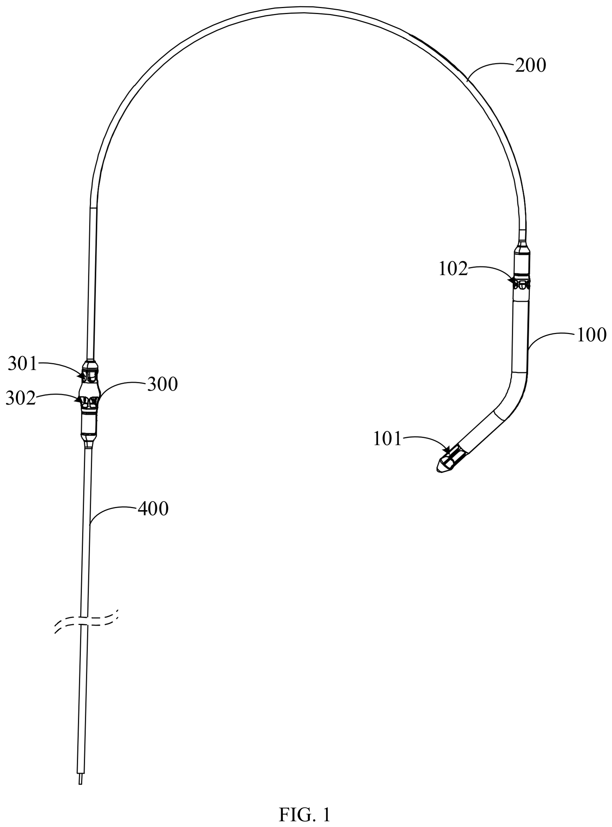

Referring to FIG. 1 to FIG. 2, the blood pump 10 includes a primary pumping device 100, a first catheter 200, a secondary pumping device 300 and a second catheter 400 which are connected in sequence. The primary pumping device 100 is provided with a first inlet 101 and a first outlet 102; the secondary pumping device 300 is provided with a second inlet 301 and a second outlet 302, and the second inlet 301 is arranged on an outer peripheral surface of the secondary pumping device 300. Specifically, a distal end of the primary pumping device 100 may be connected to a pigtail catheter (not shown in the drawings), and is supported and positioned on an inner wall of the heart through the pigtail catheter. A proximal end of the primary pumping device 100 is connected to a distal end of the first catheter 200, and a proximal end of the first catheter 200 is connected to a distal end of the secondary pumping device 300; a proximal end of the secondary pumping device 300 is connected to a distal end of the second catheter 400.

After the blood pump 10 is implanted into a patient' body, the primary pumping device 100 extends through the aorta 20, across a valve 21 and partially into the ventricle, so that the first inlet 101 of the primary pumping device 100 is located within the ventricle and the first outlet 102 of the primary pumping device 100 is located within the aorta 20; the secondary pumping device 300 and the first catheter 200 are located within the aorta 20; the second catheter 400 extends from the secondary pumping device 300 to the exterior of the patient. When the blood pump 10 is started to work, blood in the ventricle flows into the primary pumping device 100 via the first inlet 101, is accelerated by the primary pumping device 100, and then flows out of the first outlet 102 and flows to the ascending part 22 of the aorta 20, and then flows along the ascending part 22 of the aorta 20 towards the aortic arch; subsequently, the blood meets the secondary pumping device 300 and is drawn into the secondary pumping device 300 via the second inlet 301 of the secondary pumping device 300. After the secondary pumping device 300 accelerates the drawn blood again, the blood is discharged from the second outlet 302 into a descending part 23 of the aorta 20, so that the flow of the blood can be accelerated, the blood can smoothly flow through the aortic arch, and blood circulation can be speeded up.

It can be seen that, in the blood pump 10 according to the present application, the primary pumping device 100, the first catheter 200, the secondary pumping device 300 and the second catheter 400 are configured, the primary pumping device 100, the first catheter 200, the secondary pumping device 300 and the second catheter 400 are sequentially connected to each other, and the blood is accelerated at least twice by using the primary pumping device 100 and the secondary pumping device 300, so that the blood pump 10 has a dual-stage driving function, a driving force of the blood pump 10 can be effectively enhanced, and the blood flow rate pumped by the blood pump 10 can be increased. Due to the presence of the secondary pumping device 300, the secondary pumping device 300 can generate a negative pressure at the aortic arch, which causes the blood in the ascending part 22 of the aorta 20 to flow towards the descending part 23 of the aorta 20 in an accelerated manner, thereby increasing the blood flow rate.

It can be understood that, since the blood pump 10 according to the present application has the primary pumping device 100 and the secondary pumping device 300, on the premise of ensuring that total pumping power of the blood pump 10 is not less than that of a conventional blood pump 10, pumping power of a single pumping device (such as the primary pumping device 100 or the secondary pumping device 300) can be appropriately reduced to reduce an axial size of the single pumping device, so as to reduce a length of the primary pumping device 100, to facilitate implantation in the patient' body. In particular, reducing the length of the primary pumping device 100 can greatly reduce difficulty of passing the primary pumping device 100 through the aortic arch.

Since the second inlet 301 of the blood pump 10 according to the present application is arranged on the outer peripheral wall of the secondary pumping device 300, the second inlet 301 may have a large inlet area, thereby facilitating the blood in the aorta 20 to enter the secondary pumping device 300 via the second inlet 301, which can greatly increase the blood flow rate pumped by the secondary pumping device 300. In another aspect, since the proximal end of the first catheter 200 of the blood pump 10 is connected to the distal end of the secondary pumping device 300, if the second inlet 301 of the secondary pumping device 300 is also arranged at the distal end of the secondary pumping device 300, a diameter of the distal end of the secondary pumping device 300 is required to be increased, increasing the risk of the distal end of the secondary pumping device 300 colliding and damaging the blood vessel wall, and blood flow at the second inlet 301 is prone to scour a junction between the first catheter 200 and the secondary pumping device 300, which is prone to cause the first catheter 200 to fall off. Therefore, in the blood pump 10 according to the present application, the second inlet 301 is arranged in the outer peripheral wall of the secondary pumping device 300, which not only eliminate the need to increase the diameter of the distal end of the secondary pumping device 300, thereby reducing the risk of the distal end of the secondary pumping device 300 colliding and damaging the blood vessel wall, but also can reduce the scouring of the junction between the first catheter 200 and the secondary pumping device 300 by the blood flow at the second inlet 301 and prevent the proximal end of the first catheter 200 from loosening.

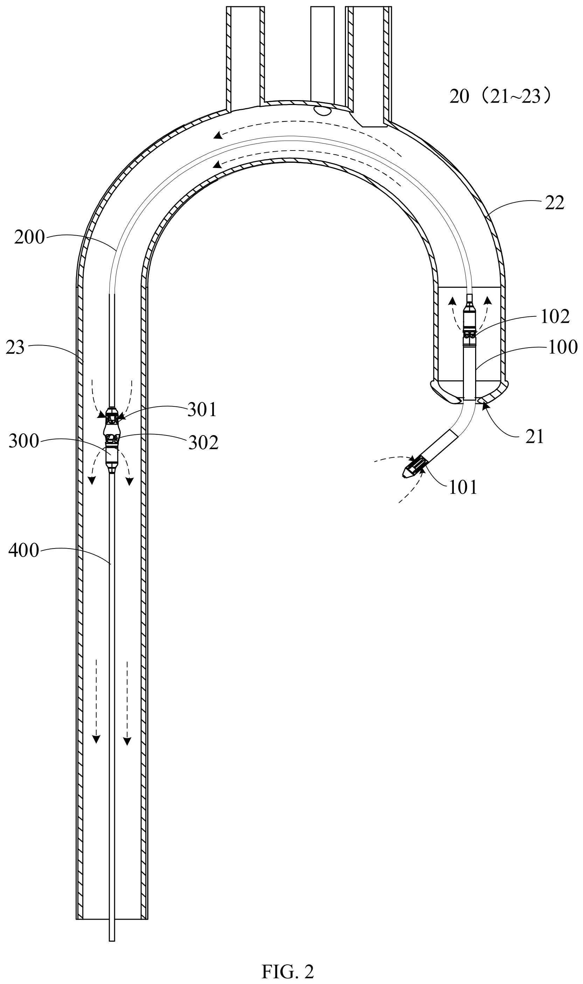

As shown in FIG. 2, in some embodiments, a length of the first catheter 200 may be selected to range from 180 mm to 300 mm. The length of the first catheter 200 within this range can enable the secondary pumping device 300 to enter the descending part 23 of the aorta 20 and be located above the renal artery to increase the blood flow rate of the renal artery. The length of the first catheter 200 may specifically be, but is not limited to, 190 mm, 200 mm, 220 mm, 250 mm, 280 mm, 290 mm, or the like.

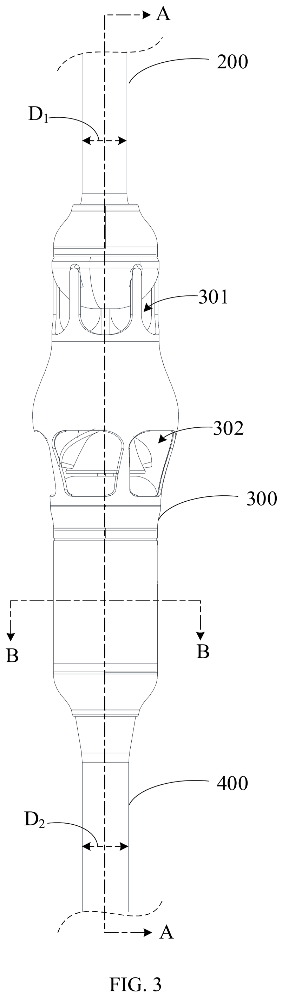

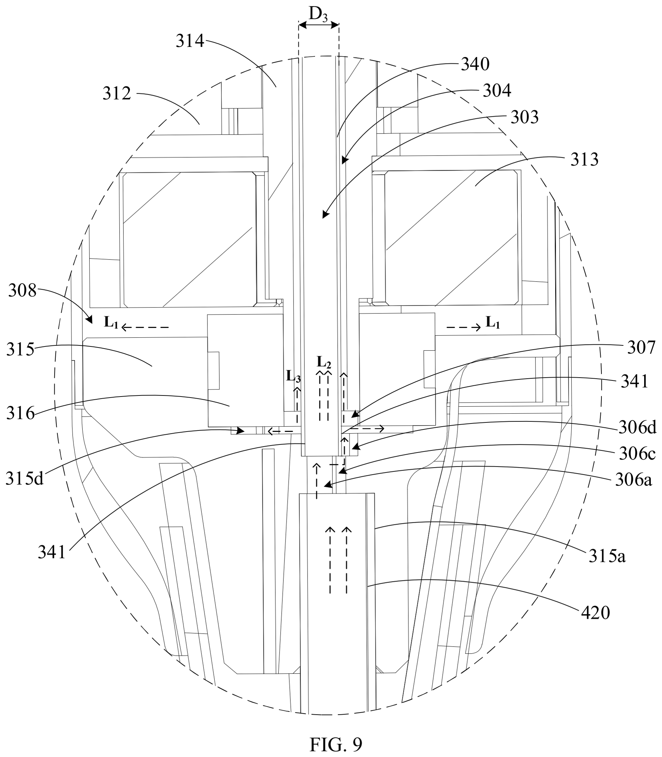

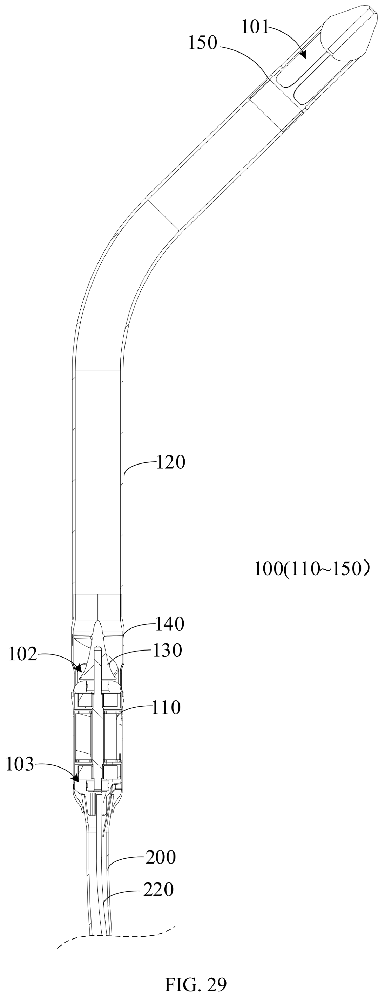

Referring to FIG. 3 and FIG. 7, in some embodiments, a first flushing flow channel 103 (see FIG. 29) is arranged in the primary pumping device 100 and configured to infuse flushing liquid (e.g., normal saline) to avoid thrombus caused by the blood entering the primary pumping device 100, and configured to dissipate heat from the primary pumping device 100. A second flushing flow channel 308 (see FIG. 9) is arranged in the secondary pumping device 300 and configured to infuse flushing liquid (e.g., normal saline) to avoid thrombus caused by the blood entering the secondary pumping device 300, and configured to dissipate heat from the secondary pumping device 300. Referring to FIG. 3 and FIG. 4, in view of this, a first flushing tube 220 for supplying the flushing liquid to the primary pumping device 100 extends through the first catheter 200; a second flushing tube 420 for supplying the flushing liquid to the secondary pumping device 300 is arranged inside the second catheter 400.

In this embodiment, in order to prevent the first flushing tube 220 from passing through the secondary pumping device 300, referring to FIG. 3, FIG. 7 and FIG. 8, an intermediate channel 303 extending through the secondary pumping device 300 in an axial direction thereof is arranged inside the secondary pumping device 300. The intermediate channel 303 communicates the first flushing tube 220 with the second flushing tube 420. That is, a distal end of the second flushing tube 420 is in communication with both the second flushing flow channel 308 and the intermediate channel 303 of the secondary pumping device 300. With such a design, a part of the flushing liquid supplied by the second flushing tube 420 can enter the second flushing flow channel 308 to be supplied to the secondary pumping device 300; another part of the flushing liquid is conveyed to the first flushing tube 220 via the intermediate channel 303 and then supplied to the primary pumping device 100 via the first flushing tube 220.

In some embodiments, a first electric wire (not shown) is further arranged in the first catheter 200, and a distal end of the first electric wire is connected to the primary pumping device 100 to provide electric energy to the primary pumping device 100. Preferably, the first electric wire and the first flushing tube 220 are arranged in the first catheter 200 without interfering with each other. In this implementation, the first electric wire extends from the proximal end to the distal end, sequentially passing through the second catheter 400, the intermediate channel 303 of the secondary pumping device 300 and the first catheter 200, before being connected to the primary pumping device 100. A second electric wire (not shown) is also arranged in the second catheter 400, and a distal end of the second electric wire is connected to the secondary pumping device 300 to provide electric energy to the secondary pumping device 300. Preferably, the second electric wire and the second flushing tube 420 are arranged in the first catheter 200 without interfering with each other. The first electric wire, the second conductive wire, and the second flushing tube 420 all need to pass through the second catheter 400, and only the first electric wire and the first flushing tube 220 need to pass through the first catheter 200. Thus, in some embodiments, referring to FIG. 3, the first catheter 200 has a first outer diameter D₁, the second catheter 400 has a second outer diameter D₂, and the first outer diameter D₁ is less than the second outer diameter D₂. With such a design, in the case where the first catheter 200 can accommodate the electric wire for the primary pumping device 100 and the first flushing tube, a radial size of the first catheter 200 can be kept small, and the first catheter 200 is flexible, so that the two pumping devices can be conveniently guided to move in the blood vessel.

Further, the second outer diameter D₂ is less than twice the first outer diameter D₁, so that in the case where the second catheter 400 can accommodate the electric wires for the two pumping devices and the second flushing tube 420, it can avoid the outer diameter D₂ of the second catheter 400 being too large to affect its implantation in the human body. If the outer diameter of the second catheter 400 exceeds twice the outer diameter D₁ of the first catheter 200, it may result in an excessively large radial size of the second catheter 400, excessive rigidity of the second catheter 400, difficulty in bending the second catheter 400 to adapt to a shape of the blood vessel, which may lead to significant implantation difficulties.

It is worth mentioning that if the first catheter 200 extends through the secondary pumping device 300, there may be several drawbacks: (1) the diameter of the first catheter 200 needs to be reduced, it is difficult for an inner cavity of the first catheter 200 to accommodate the electric wire for the primary pumping device 100 and the first flushing tube 220, the first catheter 200 may have excessively low strength, making it difficult to manipulate and guide the secondary pumping device 300 to pass through the blood vessel 200 into the heart; (2) if the diameter of the first catheter 200 is not reduced, the diameter of the secondary pumping device 300 needs to be increased, which results in an increase in a volume of the secondary pumping device 300, thereby increasing the difficulty of implantation in the human body; (3) the first catheter 200 is prone to interfere with internal members of the secondary pumping device 300 during passing through the secondary pumping device 300, and a gap between an outer periphery of the first catheter 200 and the secondary pumping device 300 is difficult to seal, allowing the blood to easily infiltrate, which may lead to thrombotic incidents, or the like.

Compared with the case where arranging the first catheter 200 to pass through as described above, in the present application, the proximal end of the first catheter 200 is connected only to the distal end of the secondary pumping device 300, and the flushing liquid or the electric wire is conveyed through the intermediate channel 303 inside the secondary pumping device 300, so that at least one of the above three drawbacks can be overcome, the diameter of the first catheter 200 is not required to be reduced, the diameter of the secondary pumping device 300 is not increased, and therefore, the secondary pumping device 300 has a small volume, thereby reducing the implantation difficulty.

Referring to FIG. 3 to FIG. 5, in some embodiments, in order to facilitate the connection of the first catheter 200 to the distal end of the secondary pumping device 300, the distal end of the secondary pumping device 300 is provided with a connecting end 324; the proximal end of the first catheter 200 is provided with a first sleeving portion 210, and the first sleeving portion 210 is sleeved on an outer peripheral surface of the connecting end 324 and is fixedly connected to the connecting end 324.

Optionally, the secondary pumping device 300 includes a secondary cannula 320. The secondary cannula 320 is configured to have a shape with a large diameter in the middle part and two small diameters at two ends. The middle portion with a large diameter can accommodate the secondary impeller 330 with a large diameter. The secondary cannula 320 is provided with the second inlet 301 and the second outlet 302; a proximal end portion of the secondary cannula 320 is connected to the second catheter 400, and a distal end portion of the secondary cannula 320 is provided with the connecting end 324. The second inlet 301 is adjacent to the connecting end 324. The second inlet 301 has a plurality of outlet holes. A connecting post 325 is formed between every two adjacent outlet holes, and a tip end of each connecting post 325 is fixedly connected to the connecting end 324. The first sleeving portion 210 is sleeved on the outer peripheral surface of the connecting end 324. The first sleeving portion 210 and the outer peripheral surface realize surfacesurface attaching and contact, so that the proximal end of the first catheter 200 can be effectively sealed to prevent the blood from infiltrating into the first catheter 200.

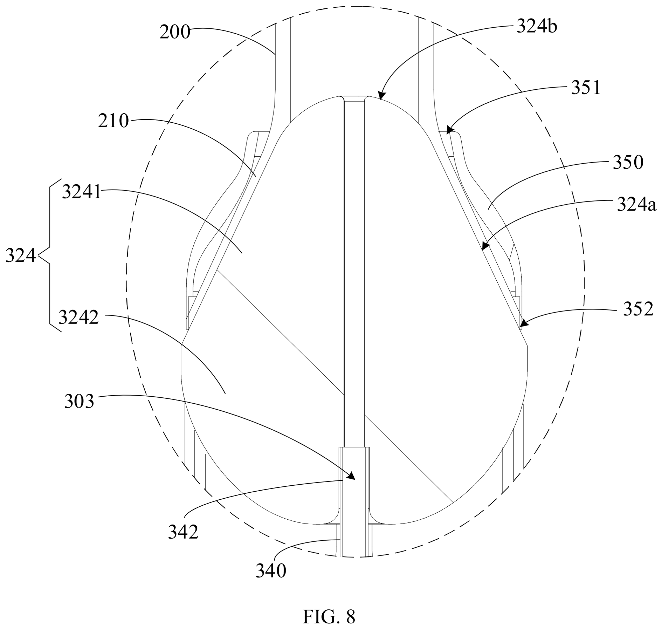

Referring to FIG. 4 to FIG. 6, in some embodiments, the first sleeving portion 210 is configured to have a horn shape, and an inner diameter of the first sleeving portion 210 is gradually increased in a direction from the first catheter 200 to the secondary pumping device 300; referring to FIG. 7 and FIG. 8, the connecting end 324 includes a first variable-diameter peripheral surface 324a, and the first variable-diameter peripheral surface 324a is configured to conform to a shape of an inner peripheral surface of the first sleeving portion 210, so as to adapt to and be coupled to the first sleeving portion 210.

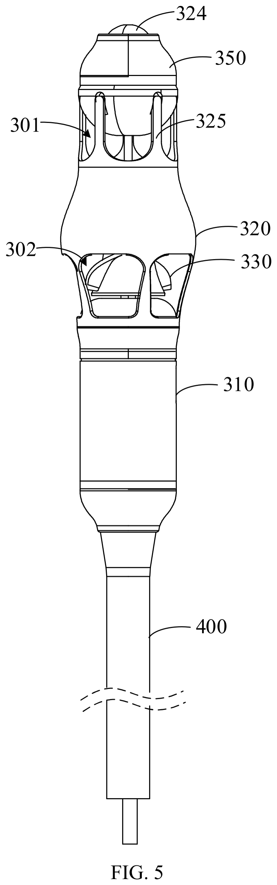

Referring to FIG. 5 to FIG. 7, specifically, the connecting end 324 includes a tapered portion 3241 and a spherical portion 3242; the spherical portion 3242 is configured to guide the flow to the second inlet 301; the tapered portion 3241 has a structure with a conical or truncated cone shape, and the tapered portion 3241 is fixedly connected to the first catheter 200. The first variable-diameter peripheral surface 324a is formed on an outer peripheral surface of the tapered portion 3241 to adapt to the first sleeving portion 210 for coupling to it. The first sleeving portion 210 is sleeved outside the first variable-diameter peripheral surface 324a, and the inner peripheral surface of the first sleeving portion 210 is connected to the first variable-diameter peripheral surface 324a. In this implementation, the first sleeving portion 210 is fixed to the first variable-diameter peripheral surface 324a by adhesive. In the direction from the secondary pumping device 300 to the first catheter 200, the attaching degree is increasingly tighter, so that the sealing performance at the proximal end of the first catheter 200 can be significantly improved.

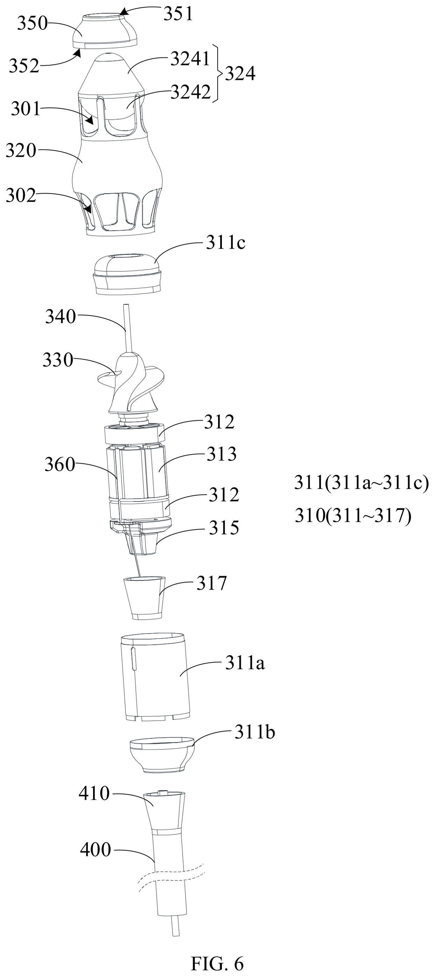

Referring to FIG. 6 to FIG. 8, in some embodiments, the proximal end of the first catheter 200 is further provided with a fixing cover 350. The fixing cover 350 is in an annular structure and is sleeved on an outer circumference of the first sleeving portion 210. The fixing cover 350 has a reduced-diameter opening 351 and an expanded-diameter opening 352. A periphery of the expanded-diameter opening 352 is fixedly connected to the connecting end 324, a periphery of the reduced-diameter opening 351 is sleeved on the first catheter 200, and a diameter of the reduced-diameter opening 351 is less than that of the first sleeving portion 210. When the fixing cover 350 connects the secondary pumping device 300 to the first catheter 200, the fixing cover 350 is sleeved on the outer periphery of the first sleeving portion 210, the first sleeving portion 210 is sleeved outside the first variable-diameter peripheral surface 324a, and the fixing cover 350 is fixedly connected to the first variable-diameter peripheral surface 324a, thereby reinforcing the connection between the first catheter 200 and the secondary pumping device 300. In this implementation, the fixing cover 350 is made of a metal material, the periphery of the expanded-diameter opening 352 of the fixing cover 350 is welded to the connecting end 324, and an outer peripheral surface of the fixing cover 350 is connected to the outer peripheral surface of the connecting end 324 in a smooth transition manner.

In some embodiments, a joint of the tapered portion 3241 and the spherical portion 3242 of the connecting end 324 is a junction having a diameter that is the maximum diameter of the spherical portion. The junction is connected to a distal end of the connecting post 325, so that the spherical portion 3242 extends into the secondary cannula 320 to direct the blood to flow in wall-adherent motion and enter the secondary cannula 320 via the second inlet 301. Preferably, the spherical portion 3242 has a hemispherical structure.

Further, a distal end surface of the tapered portion 3241 is configured as a spherical surface 324b, and a distal end of the tapered portion 3241 extends through the first sleeving portion 210 and is embedded in the inner cavity of the first catheter 200. Specifically, the distal end of the tapered portion 3241 is in interference fit with the first catheter 200. By configuring the distal end surface of the tapered portion 3241 as the spherical surface 324b, when the tapered portion 3241 is inserted into the sleeving portion 210, an external force may be used to drive the spherical surface 324b of the distal end of the tapered portion 3241 to abut against an inner wall surface of the sleeving portion 210, so as to expand the sleeving portion 210 without damaging the first sleeving portion 210, and then, the distal end of the tapered portion 3241 extends through the first sleeving portion 210 to be embedded into the inner cavity of the first catheter 200, so that the distal end of the tapered portion 3241 is in interference fit with a wall of the inner cavity of the first catheter 200, thereby improving tightness of the connection between the first sleeving portion 210 and the connecting end 324.

Referring to FIG. 6, in some embodiments, the second outlet 302 has a plurality of outlet holes that are distributed and spaced part in a circumferential direction along a periphery surface of the secondary cannula 320. A width of each of the outlet holes in the circumferential direction is decreased from a distal end to a proximal end.

Referring to FIG. 6 to FIG. 7, in some embodiments, the proximal end of the secondary pumping device 300 is provided with a fixing pin 315, and the fixing pin 315 is fixedly connected to the second catheter 400. The distal end of the second catheter 400 is provided with a second sleeving portion 410, and the second sleeving portion 410 is sleeved on a proximal end of the fixing pin 315. Specifically, a diameter of the second sleeving portion 410 is gradually increased from a proximal end to a distal end; an outer periphery of the fixing pin 315 is further wrapped with a positioning cover 317. An outer peripheral surface of the positioning cover 317 has a second variable-diameter peripheral surface. The second variable-diameter peripheral surface is configured to conform to a shape of an inner peripheral surface of the second sleeving portion 410, so that the second sleeving portion 410 is adapted to and coupled to the second variable-diameter peripheral surface.

Referring to FIG. 5 to FIG. 7, in some embodiments, the secondary pumping device 300 further includes the secondary impeller 330. The secondary impeller 330 is arranged in the secondary cannula 320. The secondary impeller 330 is rotatable relative to the secondary cannula 320 to drive the blood to enter the secondary cannula 320 via the second inlet 301 and be discharged from the second outlet 302. The secondary pumping device 300 further includes a secondary motor 310. The secondary motor 310 is connected to the second catheter 400 and the secondary cannula 320; the secondary cannula 320 is at least partially sleeved in the secondary motor 310; the secondary motor 310 can drive the secondary impeller 330 to rotate. Specifically, the secondary motor 310 includes a rotating shaft 314. A distal end of the rotating shaft 314 extends into the secondary cannula 320 to be connected to the secondary impeller 330. Certainly, in other embodiments, the secondary motor 310 is not necessary and the secondary impeller 330 may be connected to an extracorporeal motor by a flexible shaft extending through the second catheter 400.

Referring to FIG. 7 to FIG. 9, the intermediate channel 303 of the secondary pumping device 300 extends from the secondary motor 310 to the distal end of the secondary cannula 320. As for formation of the intermediate channel 303, various designs are possible. For example, the rotating shaft 314 of the secondary pumping device 300 may be configured as a hollow tube, so that an inner cavity of the rotating shaft 314 is used to form the intermediate channel 303; alternatively, a fixed tube 340 may be additionally provided in the secondary pumping device 300. The fixed tube 340 extends through the rotating shaft 314, and the fixed tube 340 is also configured as a hollow tube, so that an inner cavity of the fixed tube 340 can be used to form the intermediate channel 303.

Referring to FIG. 7 to FIG. 9, in some embodiments, the secondary pumping device 300 further includes the fixed tube 340. An end of the fixed tube 340 is fixed inside the secondary motor 310, and another end of the fixed tube 340 extending through the secondary impeller 330 to be fixed to the distal end of the secondary cannula 320; the secondary impeller 330 is rotatable about the fixed tube 340. The fixed tube 340 is configured as a hollow tube, so that the fixed tube 340 is internally hollow to form at least part of the intermediate channel 303.

Specifically, the secondary cannula 320 is partially sleeved on an outer periphery of the secondary motor 310, and the secondary motor 310 is connected to the secondary cannula 320 and the second catheter 400. A proximal end of the secondary motor 310 is fixedly connected to the second catheter 400; a distal end of the secondary motor 310 is fixedly connected to the proximal end of the secondary cannula 320; the distal end of the secondary cannula 320 is fixedly connected to the first catheter 200. The second inlet 301 and the second outlet 302 are both arranged in the secondary cannula 320. The secondary impeller 330 is arranged in the secondary cannula 320 and adjacent to the second outlet 302. The fixed tube 340 has a first end 341 and a second end 342, the second end 342 being away from the first end 341; the first end 341 is inserted into the secondary motor 310 and fixed to the proximal end of the secondary motor 310 (e.g., the fixing pin 315), so that the first end 341 is in communication with the second flushing tube 420 fixed to the proximal end of the secondary motor 310; the second end 342 extends out of the distal end of the secondary motor 310, and extends through the secondary impeller 330 and a lumen of the secondary cannula 320 and is fixed to the distal end (i.e., the connecting end 324) of the secondary cannula 320, so that the second end 342 is in communication with the first flushing tube 220 (see FIG. 29) within the first catheter 200.

It can be understood that a tip end of the primary pumping device 100 may be positioned in the ventricle by the pigtail catheter, and the middle portion of the primary pumping device 100 may be clamped by the valve and thus positioned. The entire secondary pumping device 300 is located within an artery. The second catheter 400 and the first catheter 200 are generally flexible, and therefore, the second catheter 400 and the first catheter 200 may not be sufficient to stably support the secondary pumping device 300. In the present application, since the fixed tube 340 is arranged in the secondary pumping device 300, the fixed tube 340 can support the secondary pumping device 300, so that the rotating shaft 314 and the secondary impeller 330 of the secondary pumping device 300 can stably rotate, thereby enhancing stability of the secondary pumping device 300. Even if the second catheter 400 and the first catheter 200 are scoured by the blood flow and sway when a flow velocity of the blood in the aorta 20 is high, the fixed tube 340 has high rigidity and is not prone to be bent and deflected along with the second catheter 400 and the first catheter 200, while the secondary impeller 330 is supported by the fixed tube 340, so that the secondary impeller 330 also does not readily undergo radial deflection.

Optionally, the fixed tube 340 is made of a metal material. The fixed tube 340 has a third outer diameter D₃ that is less than the first outer diameter D₁ of the first catheter 200. In this way, the fixed tube 340 may have a small radial size, and occupy a small radial space in the secondary pumping device 300, so that the secondary pumping device 300 has a small radial size, thereby reducing the implantation difficulty.

Referring to FIG. 6, FIG. 7 and FIG. 8, further, the secondary motor 310 includes: a housing 311; and a stator 312, a rotor 313 and the rotating shaft 314 that are mounted in the housing 311; the rotating shaft 314 is sleeved on an outer periphery of the fixed tube 340. The rotating shaft 314 extends out from the distal end of the secondary motor 310 and is fixedly connected to the secondary impeller 330. The rotating shaft 314 can rotate relative to the fixed tube 340; the rotor 313 is fixedly connected to an outer peripheral surface of the rotating shaft 314; the stator 312 can generate a magnetic field that drives the rotor 313 to rotate.

The rotating shaft 314 is configured as a hollow tube, so that the rotating shaft 314 has the inner cavity, and therefore, the rotating shaft 314 is rotatably sleeved on the outer periphery of the fixed tube 340. The distal end of the rotating shaft 314 extends out of the housing 311 of the secondary motor 310 to be fixedly connected to the secondary impeller 330, the rotor 313 is fixedly connected to an outer peripheral wall of the rotating shaft 314, and the stator 312 surrounds an outer peripheral of the rotating shaft 314. The rotor 313, the stator 312, a sensor 360, and the secondary impeller 330 are all connected to the outer peripheral of the rotating shaft 314, and the rotor 313, the sensor 360, and the stator 312 are all located inside the housing 311. Two rotors 313 are provided, and the two rotors 313 are respectively located at both ends of the stator 312 and are fixedly connected to the rotating shaft 314. The housing 311 includes a cylindrical shell 311a, a proximal cover 311b, and a distal cover 311c; the proximal cover 311b is connected to a proximal end of the cylindrical shell 311a. The distal cover 311c is connected to a distal end of the housing 311, so as to protect the rotor 313, the sensor 360 and the stator 312 and prevent the blood from entering the interior of the housing 311.

Referring to FIG. 7, FIG. 9 and FIG. 22, further, a proximal bearing 316 is arranged at the proximal end of the secondary motor 310. The proximal bearing 316 is mounted on the fixing pin 315. A proximal end of the rotating shaft 314 can be inserted into and connected to the proximal bearing 316; a proximal end of the fixed tube 340 extends through the rotating shaft 314 and the proximal bearing 316 to be fixedly connected to the fixing pin 315.

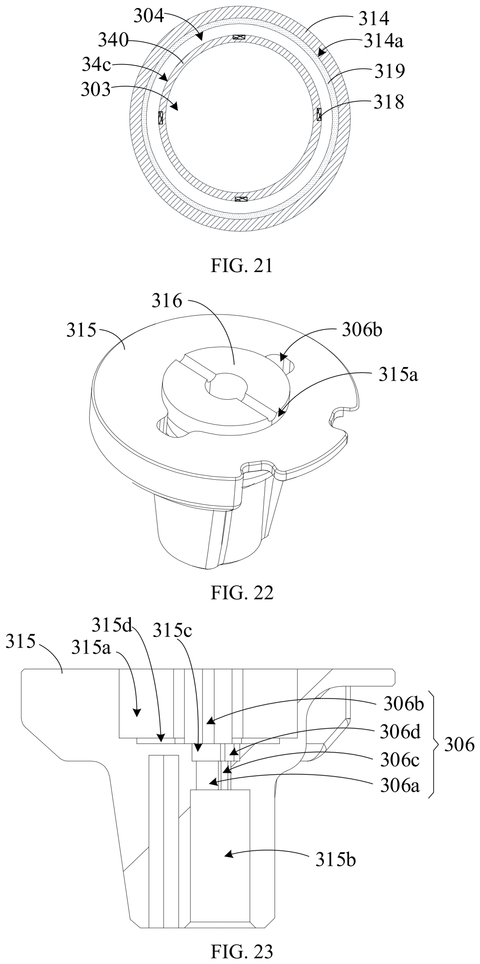

Specifically, an end of the fixing pin 315 adjacent to the second catheter 400 is provided with a first positioning hole 315b (as shown in FIG. 22 and FIG. 23), and the first positioning hole 315b is configured for insertion and fixation of the second catheter 400; another end of the fixing pin 315 (i.e., the end facing the rotating shaft 314) is provided with a mounting hole 315a and a second positioning hole 315c; the second positioning hole 315c is located on a side of the mounting hole 315a away from the rotating shaft 314. The proximal bearing 316 is embedded in the mounting hole 315a of the fixing pin 315. The proximal end of the rotating shaft 314 is inserted into and connected to the proximal bearing 316, so that the rotating shaft 314 is rotatable. The proximal end (i.e., the first end 341) of the fixed tube 340 extends out of the proximal end of the rotating shaft 314, and extends through the proximal bearing 316 to be inserted into the second positioning hole 315c, so that the first end 341 of the fixed tube 340 is fixed into the second positioning hole 315c.

Referring to FIG. 6 to FIG. 8 and FIG. 15, further, the connecting end 324 is arranged at the distal end of the secondary cannula 320 for connecting and fixing the first catheter 200; a distal end of the fixed tube 340 extends through the rotating shaft 314 and the secondary impeller 330 and is fixedly connected to the connecting end 324. That is, the distal end (i.e., the second end 342) of the fixed tube 340 is inserted into and fixed to the connecting end 324. The connecting end 324 is provided with a communication hole extending therethrough. The communication hole extends through the tapered portion 3241 and the spherical portion 3242 of the connecting end 324 in an axial direction of the secondary cannula 320. The second end 342 of the fixed tube 340 is inserted and fixed in the communication hole.

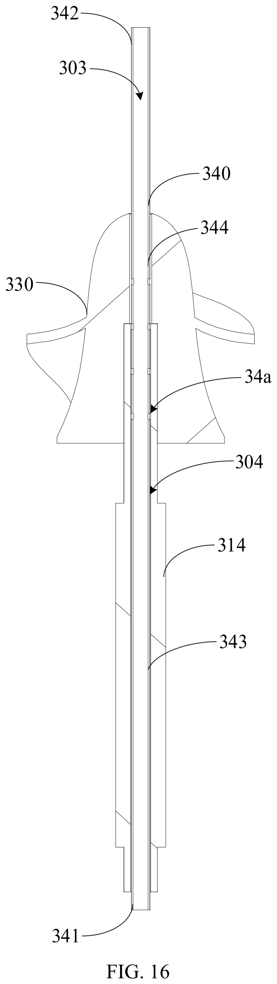

Referring to FIG. 16 and FIG. 21, optionally, at least one of an inner peripheral surface 314a of the rotating shaft 314 and an outer peripheral surface 34c of the fixed tube 340 is made of a ceramic material or a metal material, so as to reduce a friction coefficient between the rotating shaft 314 and the fixed tube 340 and reduce a friction force during the rotating shaft 314 and the fixed tube 340 rotating relative to each other.

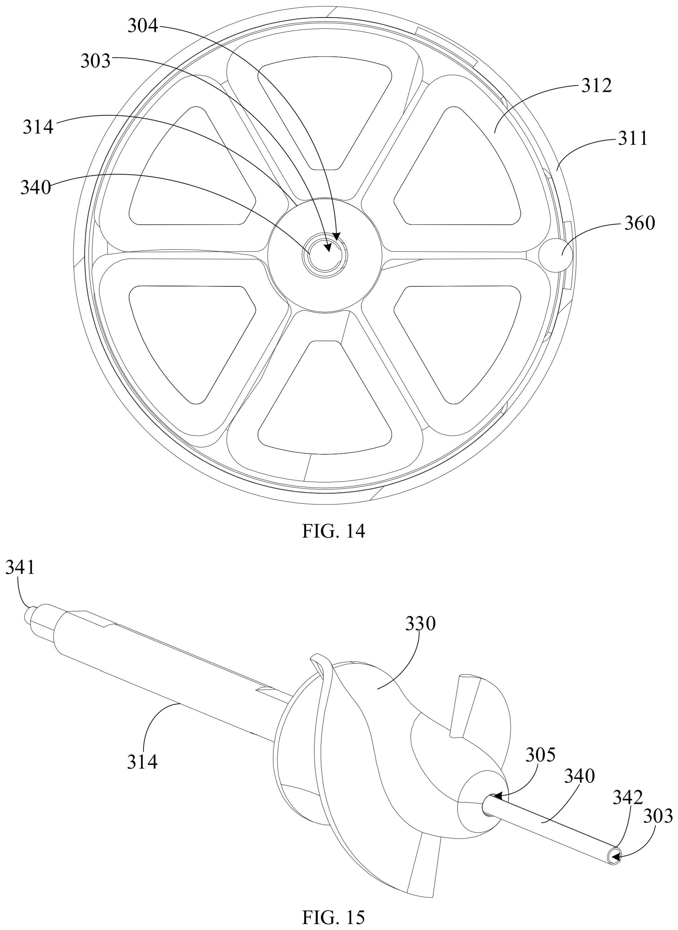

Referring to FIG. 7, FIG. 10, FIG. 18 and FIG. 21, further optionally, a first gap 304 (see FIG. 14 together) is formed between the inner peripheral surface of the rotating shaft 314 and the outer peripheral surface of the fixed tube 340. The first gap 304 is in communication with the second flushing tube 420 and the lumen of the secondary cannula 320. The secondary impeller 330 is provided with a through hole 331. The through hole 331 extends through the secondary impeller 330 in an axial direction thereof; the rotating shaft 314 is inserted and fixed to a proximal part of the through hole 331, and the fixed tube 340 extends out of a distal part of the through hole 331. A second gap 305 is formed between the outer peripheral surface of the fixed tube 340 and an inner peripheral surface of the distal part of the through hole 331. The second gap 305 is in communication with the first gap 304 and the lumen of the secondary cannula 320.

With such a design, a part of the flushing liquid in the second flushing tube 420 can enter the first gap 304 and be discharged into the lumen of the secondary cannula 320 through the second gap 305. In this way, on the one hand, the inner peripheral surface of the rotating shaft 314 and the outer peripheral surface of the fixed tube 340 can be lubricated, reducing the friction coefficient between the rotating shaft 314 and the fixed tube 340 and reducing the friction force during the rotating shaft 314 and the fixed tube 340 rotating relative to each other; on the other hand, heat generated by friction can be taken away to dissipate heat from the rotating shaft 314 and the fixed tube 340; blood within the lumen of the secondary cannula 320 is prevented from entering the secondary motor 310 via the through hole of the secondary impeller 330. Certainly, the rotating shaft 314 can be suspended relative to the fixed tube 340 by liquid, so as to reduce the friction between the rotating shaft 314 and the fixed tube 340.

Referring to FIG. 7, FIG. 18 and FIG. 21, in addition, in other embodiments, one of the inner peripheral surface 314a of the rotating shaft 314 and the outer peripheral surface 34c of the fixed tube 340 is provided with a magnetic ring 319, another of the inner peripheral surface 314a of the rotating shaft 314 and the outer peripheral surface 34c of the fixed tube 340 is provided with a magnet 318. The magnetic ring 319 and the magnet 318 repel each other, so that the inner peripheral surface 314a of the rotating shaft 314 is suspended relative to the outer peripheral surface 34c of the fixed tube 340. For example, the magnetic ring 319 may be embedded in one of the inner peripheral surface 314a of the rotating shaft 314 and the outer peripheral surface 34c of the fixed tube 340, the magnet 318 may be arranged on the other of the inner peripheral surface 314a of the rotating shaft 314 and the outer peripheral surface 34c of the fixed tube 340; and the magnetic ring 319 and the magnet 318 repel each other to generate a repulsive force, so that the rotating shaft 314 is suspended relative to the fixed tube 340, and the rotating shaft 314 and the fixed tube 340 do not contact each other, thereby reducing the friction between the rotating shaft 314 and the fixed tube 340 during rotation. It can be understood that the magnetic ring 319 and the magnet 318 are not necessary.

In some embodiments, the difference from the above embodiments lies in that the secondary motor 310 may not include the rotating shaft 314, that is, the secondary motor 310 is a shaftless motor. Specifically, the secondary motor 310 includes the housing 311, the stator 312 mounted in the housing 311, and the rotor 313. The rotor 313 is connected to the secondary impeller 330. The stator 312 is capable of generating a magnetic field that drives the rotor 313 to rotate. That is, the rotor 313 of the secondary motor 310 is directly connected to the secondary impeller 330. That is, the rotor 313 is arranged on the secondary impeller 330, and the stator 312 of the secondary motor 310 drives the rotor 313 of the secondary impeller 330 to rotate, so that the rotor 313 drives the secondary impeller 330 to rotate around the fixed tube 340.

Certainly, the manner of forming the intermediate channel 303 is not limited thereto. For example, in another implantation, the secondary pumping device 300 does not include the fixed tube 340, and the inner cavity of the rotating shaft 314 is directly taken as a part of the intermediate channel 303. Specifically, the secondary pumping device 300 includes the secondary motor 310, the secondary cannula 320, and the secondary impeller 330 arranged in the secondary cannula 320. The secondary motor 310 includes the rotating shaft 314. An end of the rotating shaft 314 is rotatably inserted into the secondary motor 310, another end of the rotating shaft 314 extends through the secondary impeller 330 to be rotatably connected to the distal end of the secondary cannula 320. The rotating shaft 314 is configured to drive the secondary impeller 330 to rotate. The rotating shaft 314 is internally hollow to form at least part of the intermediate channel 303. While the secondary impeller 330 is driven by the rotating shaft 314 to rotate, the intermediate channel 303 in the rotating shaft 314 may carry the flushing liquid to convey it to the first flushing tube 220 in the first catheter 200. For another example, in other implementations, the fixed tube 340 may also be configured as a solid rod, and a length of the rotating shaft 314 is increased, so that the distal end of the rotating shaft 314 can be rotatably mounted to the connecting end 324. The first gap 304 formed between the outer peripheral surface of the rotating shaft 314 and the inner peripheral surface of the fixed tube 340 may serve as the intermediate channel 303. With such a design, a part of the flushing liquid supplied by the second flushing tube 420 can be conveyed to the first flushing tube 220, the distal end of the first gap 304 is not exposed to a distal end of the secondary impeller 330, so that the blood in the secondary cannula 320 is not easy to enter the secondary motor 310 via the first gap 304. The solid fixed tube 340 has large strength, so that the diameter of the fixed tube 340 is appropriately reduced while sufficient strength of the fixed tube 340 is ensured, thereby reducing the radial space in the secondary pumping device 300 occupied by the fixed tube 340, and then reducing the radial size of the secondary pumping device 300.

Referring to FIG. 7, FIG. 10 and FIG. 15, in some embodiments, since the fixed tube 340 extends through the rotating shaft 314, the fixed tube 340 includes a first middle section 343 extending through the rotating shaft 314. Optionally, the first gap 304 is formed between an outer peripheral surface of the first middle section 343 and the inner peripheral surface of the rotating shaft 314, so that two ends of the first gap 304 are in communication with the second flushing tube 420 and the lumen of the secondary cannula 320 respectively. In this way, a part of the flushing liquid supplied by the second flushing tube 420 can be partially diverted into the first gap 304 and discharged into the lumen of the secondary cannula 320 along the first gap 304.

Based on the above arrangement, on the one hand, the inner peripheral surface of the rotating shaft 314 and the outer peripheral surface of the fixed tube 340 can be lubricated to reduce the friction coefficient between the rotating shaft 314 and the fixed tube 340 and reduce the friction force during the rotating shaft 314 and the fixed tube 340 rotating relative to each other; on the other hand, the heat generated by the friction can be taken away to dissipate heat from the rotating shaft 314 and the fixed tube 340; the blood within the lumen of the secondary cannula 320 is prevented from entering the secondary motor 310 via the through hole 331 of the secondary impeller 330. Certainly, the rotating shaft 314 can be suspended relative to the fixed tube 340 by liquid, so as to reduce the friction between the rotating shaft 314 and the fixed tube 340.

As shown in FIG. 7, FIG. 22 and FIG. 23, the end of the fixing pin 315 adjacent to the second catheter 400 is provided with the first positioning hole 315b. The second flushing tube 420 is inserted into the first positioning hole 315b. The flushing liquid supplied by the second flushing tube 420 firstly enters the first positioning hole 315b, and then is divided into three branches through the first positioning hole 315b and a shunt channel 306 on the fixing pin 315. The fluid of the three branches respectively enters the second flushing flow channel 308, the intermediate channel 303 and the first gap 304.

Referring to FIG. 7, FIG. 10 and FIG. 17, further, the through hole 331 arranged in the secondary impeller 330 extends through the secondary impeller 330 in the axial direction of the secondary impeller 330; the distal end of the rotating shaft 314 is inserted and fixed to a proximal part (i.e., a large diameter section 331a) of the through hole 331, and the fixed tube 340 extends out of a distal part (i.e., a small diameter section 331b) of the through hole 331. The fixed tube 340 further includes a second middle section 344 extending through the through hole 331; the second gap 305 is formed between an outer peripheral surface of the second middle section 344 and an inner peripheral surface of the through hole 331, and the second gap 305 is in communication with the first gap 304 and the lumen of the secondary cannula 320. In this way, the flushing liquid in the second flushing tube 420 may partially enter the first gap 304 and be discharged into the lumen of the secondary cannula 320 via the second gap 305, thereby preventing a distal end of the second gap 305 from entering the impeller and the secondary motor 310 and reducing the risk of generating thrombus.

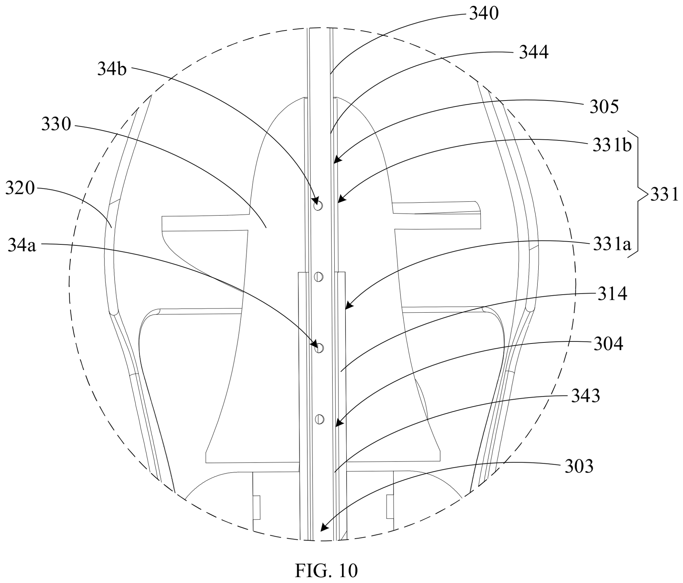

Referring to FIG. 7 and FIG. 10, in some embodiments, a side wall of the fixed tube 340 may further be provided with a draining hole. The draining hole is configured to be in communication with the intermediate channel 303 and the first gap 304 or the second gap 305, so that the fluid in the intermediate channel 303 may be partially drained to the first gap 304 or the second gap 305, which can increase the flow rate and the flow velocity in the first gap 304 or the second gap 305, so that, resistance generated when the blood in the lumen of the secondary cannula 320 enters the secondary impeller 330 and the secondary motor 310 via the second gap 305 is increased, thereby more effectively preventing generation of the thrombus.

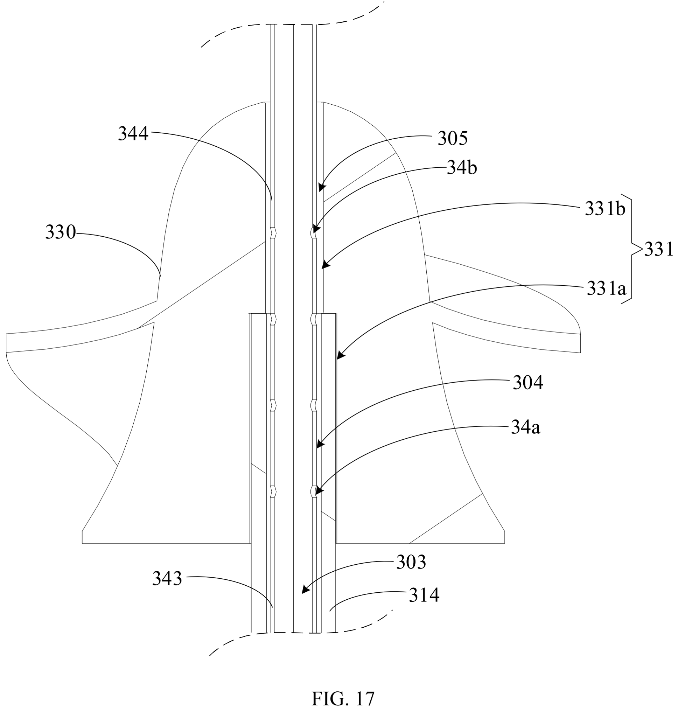

Optionally, a first draining hole 34a (see FIG. 17 to FIG. 19) extends through a side wall of the first middle section 343, and the first draining hole 34a is in communication with the first gap 304; and/or a second draining hole 34b (see FIG. 17 to FIG. 19) extends through a side wall of the second middle section 344, and the second draining hole 34b is in communication with the second gap 305. That is, the fixed tube 340 may be provided with only one of the first draining hole 34a and the second draining hole 34b, or may be provided with both the first draining hole 34a and the second draining hole 34b. One or more first draining holes 34a may be provided; one or more second draining holes 34b may be provided.

Specifically, in this embodiment, a plurality of first draining holes 34a extend through the side wall of the first middle section 343; at least some of the first draining holes 34a are arranged in an axial direction of the first middle section 343. With such an arrangement, the fluid in the first middle section 343 can be gradually drained in a flowing direction thereof, which helps to push the fluid in the first gap 304 to flow towards the second gap 305, and accelerates the discharge of the fluid.

Certainly, in other embodiments, at least some of the first draining holes 34a may be arranged in a circumferential direction of the first middle section 343. With such an arrangement, the fluid in the first middle section 343 can be discharged radially along an outer circumference of the first middle section 343 when flowing out from the at least some draining holes, so that the flushing liquid can have a more uniform flow velocity at positions of the periphery surface of the first gap 304, which is more beneficial to preventing backflow of the blood to the secondary motor 310 via the first gap 304.

Referring to FIG. 7, FIG. 17 and FIG. 19, in some implementations, a plurality of second draining holes 34b extend through the side wall of the second middle section 344. At least some of the second draining holes 34b are arranged in an axial direction of the second middle section 344. With such an arrangement, the fluid in the second middle section 344 can be gradually drained in a flowing direction thereof, which helps to push the fluid in the second gap 305 to be discharged towards the lumen of the secondary cannula 320, and accelerates the discharge of the fluid.

Certainly, in other embodiments, at least some of the second draining holes 34b may be arranged in a circumferential direction of the second middle section 344. With such an arrangement, the fluid in the second middle section 344 can be discharged radially along an outer circumference of the second middle section 344 when flowing out from the at least some draining holes, so that the flushing liquid can have a more uniform flow velocity at positions of the periphery surface of the second gap 305, which is more beneficial to preventing backflow of the blood to the first gap 304 via the second gap 305.

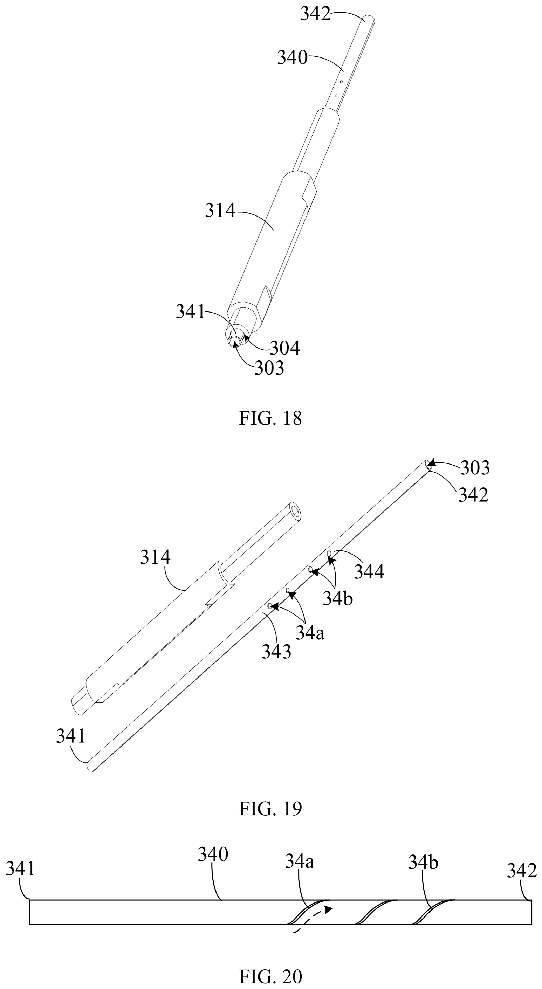

In some implementations, at least one of the first draining hole 34a and the second draining hole 34b is spirally arranged in and surrounds a circumferential wall of the fixed tube 340, and extends in an axial direction of the fixed tube 340. As shown in FIG. 20, taking the first draining hole 34a spirally arranged in and surrounding the circumferential wall of the fixed tube 340 as an example, when the rotating shaft 314 rotates, a driving force in a spiral direction is applied to the fluid discharged from the first draining hole 34a. Since the fluid discharged from the first draining hole 34a also has a spiral surrounding tendency, under the driving force generated by the rotation of the rotating shaft 314, the fluid discharged from the first draining hole 34a to the first gap 304 may spirally flow to the second gap 305 (as shown by the dotted arrow in FIG. 20),which can increase the flow rate and the flow velocity in the first gap 304, so that the resistance generated when the blood in the lumen of the secondary cannula 320 enters the impeller and the secondary motor 310 via the second gap 305 is greater, thereby more effectively preventing the generation of the thrombus.

Referring to FIG. 7, FIG. 9 and FIG. 23, in some embodiments, the intermediate channel 303 of the secondary pumping device 300 extends through the secondary motor 310 and extends to the distal end of the secondary cannula 320. The first flushing flow channel 103 is arranged inside a primary motor 110 of the primary pumping device 100. The proximal end of the first flushing flow channel 103 is in communication with the distal end of the first flushing tube 220 inside the first catheter 200, the proximal end of the first flushing tube 220 is in communication with the distal end of the intermediate channel 303, and the proximal end of the intermediate channel 303 is in communication with the second flushing tube 420 inside the second catheter 400, so that the flushing liquid conveyed by the second flushing tube 420 can partially flow into the first flushing flow channel 103 of the primary motor 110 through the intermediate channel 303. The second flushing flow channel 308 is arranged inside the secondary motor 310 of the secondary pumping device 300. The proximal end of the second flushing flow channel 308 is in communication with the second flushing tube 420 inside the second catheter 400, so that the flushing liquid conveyed by the second flushing tube 420 can also partially enter the second flushing flow channel 308 of the secondary motor 310. That is, the second flushing tube 420 is in communication with both the intermediate channel 303 and the second flushing flow channel 308 of the secondary pumping device 300.

Based on this, in order to provide a communication between the second flushing tube 420 and both the intermediate channel 303 and the second flushing flow channel 308 of the secondary pumping device 300, in this embodiment, the secondary motor 310 is further provided with the shunt channel 306. The shunt channel 306 is located at the proximal end of the secondary motor 310; the second flushing tube 420 is connected to the shunt channel 306 to distribute the flow to the second flushing flow channel 308 and the intermediate channel 303 by the shunt channel 306.

That is, the distal end of the second flushing tube 420 is connected to the shunt channel 306, and the shunt channel 306 is in communication with both the second flushing flow channel 308 and the intermediate channel 303 of the secondary pumping device 300. With such a design, the flushing liquid supplied by the second flushing tube 420 can be divided by the shunt channel 306 into at least two branches: a first branch L₁ and a second branch L₂. The first branch L₁ flows towards the second flushing flow channel 308 to be supplied to the secondary pumping device 300; the second branch L₂ is conveyed to the first flushing tube 220 via the intermediate channel 303 and then supplied to the primary pumping device 100 via the first flushing tube 220. As such, compared with the aforementioned manner of extending the first catheter 200 through the secondary pumping device 300, in the present application, the first catheter 200 and the proximal end of the first flushing tube 220 thereof can be connected only to the distal end of the secondary pumping device 300, and the flushing liquid is conveyed through the intermediate channel 303 inside the secondary pumping device 300, so that the first catheter 200 is not required to pass through the secondary pumping device 300, the diameter of the first catheter 200 is not required to be reduced or the diameter of the secondary pumping device 300 is not required to be increased, and as such, the secondary pumping device 300 has a small volume, thereby reducing the difficulty of implanting the blood pump.

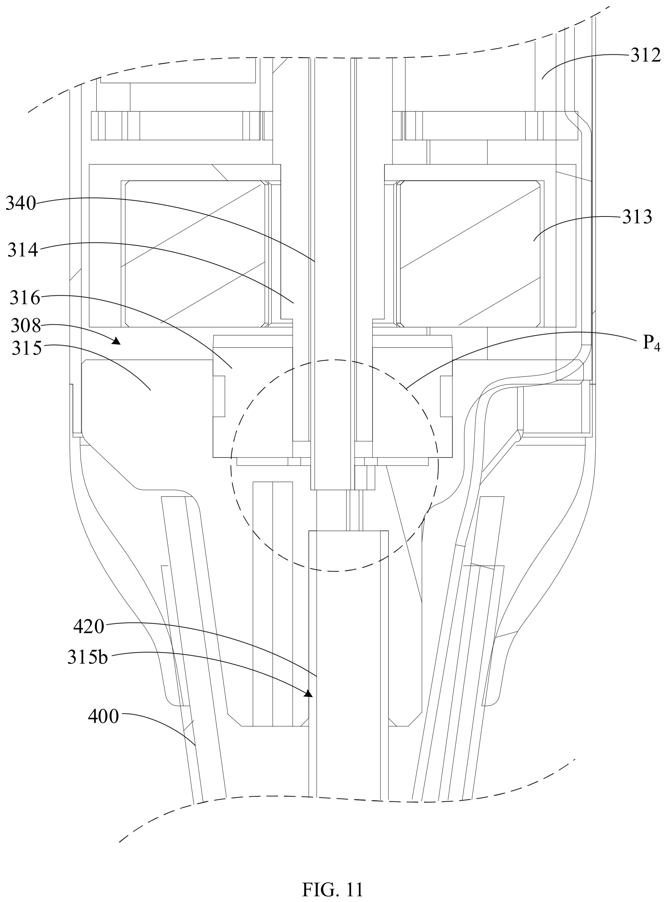

Referring to FIG. 7, FIG. 11 and FIG. 12, the first gap 304 between the inner peripheral surface of the rotating shaft 314 and the outer peripheral surface of the fixed tube 340 is in communication with the shunt channel 306 and the lumen of the secondary cannula 320. That is, the first gap 304 is in communication with the second flushing tube 420 through the shunt channel 306. Therefore, the flushing liquid supplied by the second flushing tube 420 is divided by the shunt channel 306 and then has a third branch L₃shunt channel. The third branch L₃ flows towards the first gap 304 and is discharged into the lumen of the secondary cannula 320 via the first gap 304.

The flushing liquid flows into the first gap 304 by means of the third branch L₃, so that the inner peripheral surface of the rotating shaft 314 and the outer peripheral surface of the fixed tube 340 can be lubricated, the friction coefficient between the rotating shaft 314 and the fixed tube 340 can be reduced, and the friction force generated during the rotating shaft 314 and the fixed tube 340 rotating relative to each other can be reduced. In another aspect, the heat generated by the friction between the rotating shaft 314 and the fixed tube 340 can be taken away to dissipate heat from the rotating shaft 314 and the fixed tube 340, and the blood within the lumen of the secondary cannula 320 is prevented from entering the secondary motor 310 via the through hole 331 of the secondary impeller 330. Certainly, the rotating shaft 314 can be suspended relative to the fixed tube 340 by liquid, which reduces the friction between the rotating shaft 314 and the fixed tube 340.

Referring to FIG. 9, FIG. 11 and FIG. 12, considering that the first gap 304 is small, when the rotating shaft 314 rotates, the fluid in the shunt channel 306 is easily be dispersed and flow radially by a centrifugal effect caused by the rotation of the rotating shaft 314, and may not easily enter the first gap 304. Therefore, in order to improve this situation, in one of the embodiments, the proximal bearing 316 is arranged at the proximal end of the secondary motor 310. The proximal bearing 316 is provided with a shaft hole. The shaft hole has a first opening 316a adjacent to the shunt channel 306. The proximal end of the rotating shaft 314 is inserted into the shaft hole. An end surface of the proximal end of the rotating shaft 314 and the first opening 316a are axially spaced apart to form a liquid inlet groove 307. The liquid inlet groove 307 is in communication with the first gap 304 and the shunt channel 306.

Specifically, the shaft hole axially extends through the proximal bearing 316, and the shaft hole has the first opening 316a and a second opening 316b; the first opening 316a is located at an end of the proximal bearing 316 facing the shunt channel 306, that is, ta an end adjacent to the second flushing tube 420; the second opening 316b is located at an end of the proximal bearing 316 facing the rotor 313. The proximal end of the rotating shaft 314 is inserted into the shaft hole via the second opening 316b, and the end surface of the proximal end of the rotating shaft 314 does not extends out of the first opening 316a, so that the end surface of the proximal end of the rotating shaft 314 is spaced apart from the first opening 316a to form the liquid inlet groove 307. The liquid inlet groove 307 is actually defined by an inner wall surface of the rotating shaft 314, the end surface of the proximal end of the rotating shaft 314 and the outer peripheral surface of the fixed tube 340.

Based on this, the fluid of the third branch L₃ can enter the liquid inlet groove 307 first, and the fluid in the liquid inlet groove 307 is rotated and compressed by a rotating force of the rotating shaft 314, so that the liquid inlet groove 307 has potential energy to spirally move towards the first gap 304, and the fluid in the liquid inlet groove 307 can enter the first gap 304 more easily.

Referring to FIG. 7, FIG. 9, FIG. 22 and FIG. 23, in some implementations, the fixing pin 315 is arranged at the proximal end of the secondary motor 310. An end of the fixing pin 315 is arranged in the first positioning hole 315b. The first positioning hole 315b is configured for insertion and fixation of the second flushing tube 420; another end of the fixing pin 315 is provided with the mounting hole 315a for mounting the proximal bearing 316. Optionally, the shunt channel 306 is arranged in the fixing pin 315 and extends from the first positioning hole 315b to the mounting hole 315a.

Reference may be made to the embodiments listed above for the intermediate channel 305, which is not repeatedly described herein. The formation manner of the shunt channel 306 can be reasonably set according to the number of the branches. In this embodiment, the shunt channel 306 includes a liquid flow hole 306a and a first shunt groove 306b. The liquid flow hole 306a is arranged between the mounting hole 315a and the first positioning hole 315b and in communication with the first positioning hole 315b, the intermediate channel 303 and the mounting hole 315a. The first shunt groove 306b is arranged in a side wall of the mounting hole 315a and in communication with the second flushing flow channel 308 and the liquid flow hole 306a. The first shunt groove 306b extends in an axial direction of the fixing pin 315.

It can be understood that the flushing liquid supplied by the second flushing tube 420 firstly enters the liquid flow hole 306a of the shunt channel 306 via the first positioning hole 315b, and then is divided into the first branch L₁ and the second branch L₂ at the liquid flow hole 306a; the first branch L₁ flows from the liquid flow hole 306a, the mounting hole 315a and the first shunt groove 306b towards the second flushing flow channel 308; the second branch L₂ flows from the liquid flow hole 306a and the intermediate channel 303 towards the first flushing tube 220.

Further, the fixing pin 315 is further provided with a sunken groove 315d and the second positioning hole 315c; the sunken groove 315d is formed in a bottom wall of the mounting hole 315a and in communication with the first shunt groove 306b. The second positioning hole 315c is formed in the bottom wall of the sunken groove 315d and configured for insertion of the fixed tube 340 forming the intermediate channel 303. The shunt channel 306 further includes a second shunt groove 306c and a third shunt groove 306d; the second shunt groove 306c is arranged in a side wall of the liquid flow hole 306a; the third shunt groove 306d is arranged in a side wall of the second positioning hole 315c, and is sequentially in communication with the second shunt groove 306c and the sunken groove 315d.

It can be understood that the second shunt groove 306c and the third shunt groove 306d both extend in the axial direction of the fixing pin 315; and the second shunt groove 306c, the third shunt groove 306d and the first shunt groove 306b are arranged in the axial direction of the fixing pin 315. The second shunt groove 306c, the third shunt groove 306d and the sunken groove 315d are sequentially in communication with each other, so that the first positioning hole 315b is in communication with the mounting hole 315a. A communication with the proximal end of the first gap 304 is provided by the sunken groove 315d. A diameter of the sunken groove 315d is less than that of the mounting hole 315a. A diameter of the liquid flow hole 306a is less than that of the first positioning hole 315b and less than that of the second positioning hole 315c. Based on this, the flushing liquid supplied by the second flushing tube 420 enters the liquid flow hole 306a of the shunt channel 306 and is then divided at least into the first branch L₁, the first branch L₂, and the first branch L₃ as described below.

The first branch L₁ of the flushing liquid supplied by the second flushing tube 420 flows, via the liquid flow hole 306a, sequentially through the second shunt groove 306c, the third shunt groove 306d, the sunken groove 315d and the first shunt groove 306b, and enters the second flushing flow channel 308 of the secondary pumping device 300, and finally, is discharged from a spacing space between the distal end of the secondary motor 310 and the rotating shaft 314 into the lumen of the secondary cannula 320, and ultimately, is discharged from the second outlet 132, so as to flush the interior of the secondary pumping device 300 to avoid that the blood enters the secondary motor 310 to form the thrombus. It can be understood that within the secondary motor 310, the flushing liquid passes through at least a spacing space between the housing 311 and the rotor 313 and the stator 312, and a gap between the rotating shaft 314 and the stator 312.

The second branch L2 of the flushing liquid supplied by the second flushing tube 420 enters the intermediate channel 303 via the liquid flow hole 306a and flows into the first flushing tube 220 from the intermediate channel 303, so as to be conveyed to the first flushing flow channel 103 of the primary pumping device 100 by the first flushing tube 220, so as to flush the interior of the primary pumping device 100 to avoid that the blood enters the primary motor 110 to form the thrombus.

The third branch L₃ of the flushing liquid supplied by the second flushing tube 420 flows, via the liquid flow hole 306a, sequentially through the second shunt groove 306c, the third shunt groove 306d, the sunken groove 315d and the liquid inlet groove 307, and enters the first gap 304, and finally passes through the first gap 304 and is discharged into the lumen of the secondary cannula 320, so that the rotating shaft 314 can be lubricated, and the friction between the rotating shaft 314 and the fixed tube 340 can be reduced; in another aspect, the blood can be pushed towards the distal end of the rotating shaft 314 to avoid that the blood enters via the first gap 304 to form the thrombus.

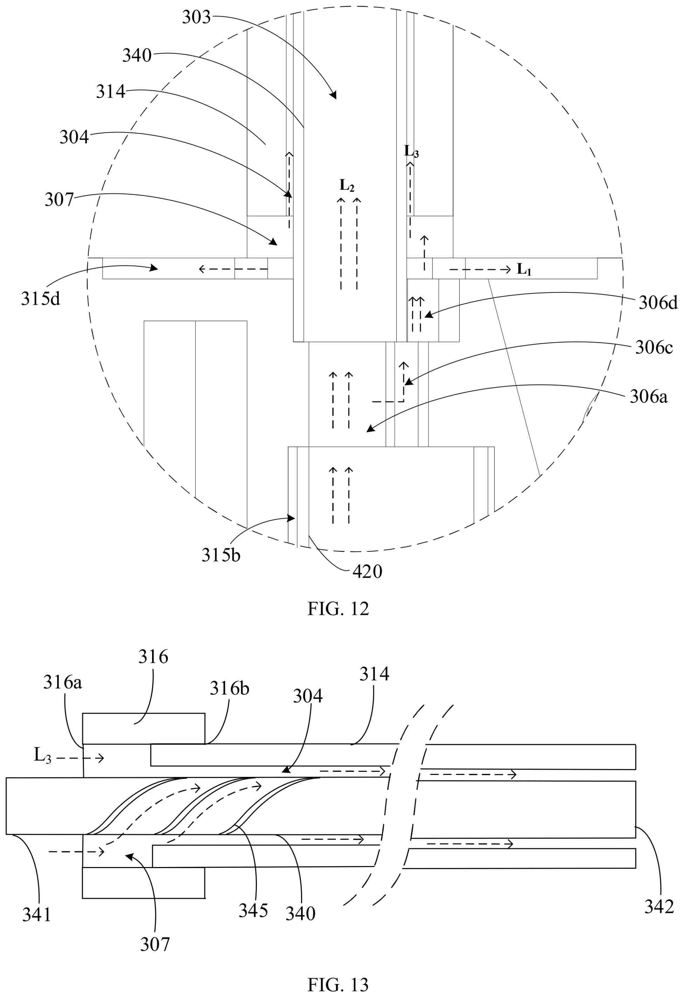

Referring to FIG. 13, in some embodiments, a flow guide structure 345 is arranged on the inner peripheral surface of the rotating shaft 314 or the outer peripheral surface of the fixed tube 340. The flow guide structure 345 is located in the first gap 304. The flow guide structure 345 extends spirally in an axial direction of the rotating shaft 314. The flow guide structure 345 may be a groove or a convex rib, and the groove or the convex rib extends spirally in the axial direction of the rotating shaft 314. When the rotating shaft 314 rotates, the fluid in the first gap 304 is rotated and compressed by the rotating force of the rotating shaft 314, and thus obtains potential energy to spirally move towards the distal end of the first gap 304, so that the fluid in the first gap 304 is accelerated to be discharged into the lumen of the secondary cannula 320, which increases the flow velocity of the fluid and the blood flow rate passing through the first gap 304, so that resistance to prevent the backflow of the blood to the secondary motor 310 via the first gap 304 is higher, and an effect of preventing the thrombus is better.

In some embodiments, the flow guide structure 345 is arranged on the outer peripheral surface of the fixed tube 340. Optionally, at least part of the flow guide structure 345 is located on the outer peripheral surface of the fixed tube 340 extending out from the proximal end of the rotating shaft 314. That is, the at least part of the flow guide structure 345 is located on the outer peripheral surface of the fixed tube 340 between the first end 341 and the first middle section 343, so that the at least part of the flow guide structure 345 can extend into the liquid inlet groove 307. With such a design, when the rotating shaft 314 rotates, the fluid of the liquid inlet groove 307 is guided by the flow guide structure 345 to spirally enter the first gap 304, so that the resistance generated when the fluid enters the first gap 304 is reduced.

In addition, further optionally, at least part of the flow guide structure 345 is located on the outer peripheral surface of the fixed tube 340 adjacent to the distal end of the rotating shaft 314. That is, the at least part of the flow guide structure 345 is arranged at a distal end of the first middle section 343 of the fixed tube 340. With such a design, when the rotating shaft 314 rotates, the fluid in the first gap 304 is guided by the flow guide structure 345 to be accelerated and discharged into the secondary cannula 320, thereby effectively preventing the blood in the secondary cannula 320 from flowing back via the first gap 304.

Referring to FIG. 7, FIG. 16 and FIG. 17, optionally, the secondary impeller 330 is further provided with the through hole 331. The through hole 331 extends through the secondary impeller 330 in the axial direction thereof; the through hole 331 includes a large diameter section 331a adjacent to the secondary motor 310, and a small diameter section 331b connected to the large diameter section 331a. The distal end of the rotating shaft 314 is embedded into the large diameter section 331a of the through hole 331, that is, the distal end of the rotating shaft 314 is inserted and fixed to the large diameter section 331a. The first middle section 343 of the fixed tube 340 is located inside the rotating shaft 314; the second middle section 344 of the fixed tube 340 is connected to the first middle section 343. The second middle section 344 extends through the small diameter section 331b. The outer peripheral surface of the second middle section 344 is spaced apart from an inner peripheral surface of the small diameter section 331b to form the second gap 305, and the second gap 305 is in communication with the first gap 304 and the lumen of the secondary cannula 320. In this way, the flushing liquid of the second flushing tube 420 may partially enter the first gap 304 and be discharged into the lumen of the secondary cannula 320 via the second gap 305, thereby avoiding that the blood enters the second gap 305 to form the thrombus, and reducing the risk of generating thrombus.

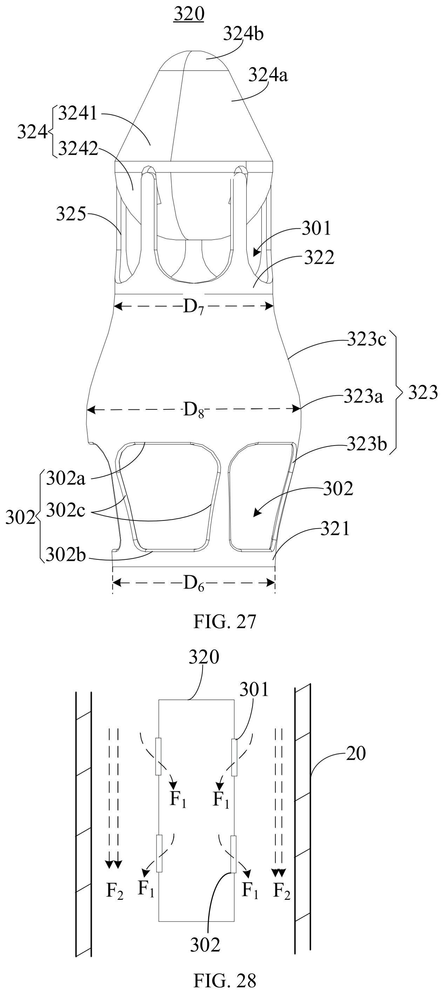

It is considered herein that the entire secondary pumping device 300 is located within the aorta 20, and the blood flows within the aorta 20 in a length direction of the aorta 20, that is, in an axial direction of the secondary pumping device 300. When the blood passes by an outer periphery of the second inlet 301 of the secondary cannula 320, a part of the blood F₁ is drawn into the secondary cannula 320 via the second inlet 301 under a suction force of the secondary pumping device 300 (as shown in FIG. 28), is accelerated within the secondary cannula 320, then flows along the secondary cannula 320 towards the second outlet 302, and finally is discharged from the second outlet 302; due to the high flow velocity of the blood, a certain part of blood F₂ inevitably fails to be drawn into the secondary cannula 320 via the second inlet 301 and thus is not accelerated by the secondary pumping device 300. Instead, this part of blood F₂ flows directly along the outer periphery of the secondary cannula 320, reducing the blood flow rate pumped by the secondary pumping device 300.

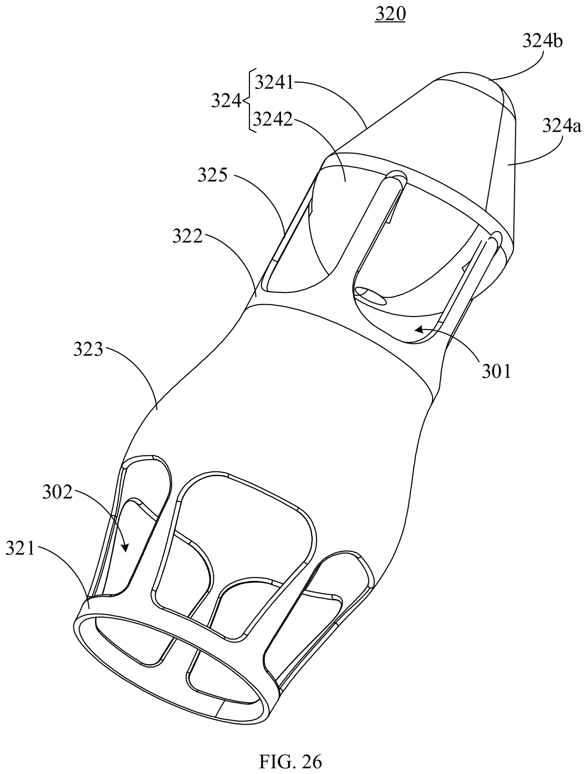

In view of the above problem, referring to FIG. 7 and FIG. 24 to FIG. 26, in order to increase the blood flow rate pumped by the secondary pumping device 300, in some embodiments, the secondary cannula 320 includes a proximal end portion 321, a distal end portion 322, and an expanded diameter portion 323; the proximal end portion 321 is adjacent to the second catheter 400, and the proximal end portion 321 is provided with the second outlet 302; the distal end portion 322 is connected to the first catheter 200, and the distal end portion 322 is provided with the second inlet 301; the expanded diameter portion 323 is located between the proximal end portion 321 and the distal end portion 322. Two ends of the expanded diameter portion 323 are smoothly and transitionally connected to the proximal end portion 321 and the distal end portion 322 respectively.

Specifically, diameters of the proximal end portion 321 and the distal end portion 322 are less than a diameter of the expanded diameter portion 323, so that the secondary cannula 320 has a shape with a large diameter in the middle part and two small diameters at two ends. The middle part with a large diameter can accommodate the secondary impeller 330 with a large diameter. The two ends of the expanded diameter portion 323 are smoothly and transitionally connected to the proximal end portion 321 and the distal end portion 322 respectively. That is, two ends of an inner wall surface of the expanded diameter portion 323 are smoothly and transitionally connected to an inner wall surface of the proximal end portion 321 and an inner wall surface of the distal end portion 322, respectively, and two ends of an outer wall surface of the expanded diameter portion 323 are also smoothly and transitionally connected to the inner wall surface of the proximal end portion 321 and the inner wall surface of the distal end portion 322, respectively. Optionally, the proximal end portion 321, the distal end portion 322, and the expanded diameter portion 323 of the secondary cannula 320 may be integrally formed.

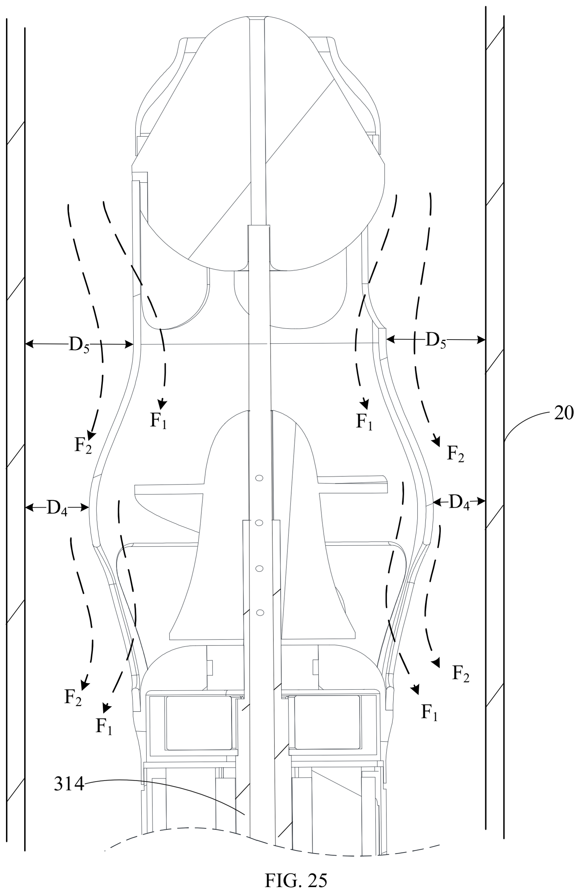

Referring to FIG. 7 and FIG. 25, since the secondary cannula 320 has the expanded diameter portion 323, after the blood pump 10 is implanted to a target site of the human body, the secondary pumping device 300 is located in the aorta 20, the expanded diameter portion 323 of the secondary cannula 320 and an inner wall surface of the aorta 20 have a first distance D₄ therebetween, the distal end portion 322 of the secondary cannula 320 and the inner wall surface of the aorta 20 have a second distance D₅ therebetween, and the first distance D₄ is less than the second distance D₅. In this way, when the secondary pumping device 300 is working, the blood F₂ after flowing along the outer periphery of the secondary cannula 320 will be throttled at the first distance D₄, and the throttled part of the blood can be drawn into the second inlet 301. This effectively increase the blood flow rate drawn into the second inlet 301, and thus increase the blood flow rate pumped by the secondary cannula 320. In other words, with the arrangement of the expanded diameter portion 323 of the secondary cannula 320, the flow rate of the blood F₂ directly flowing along the outer periphery of the secondary cannula 320 may be reduced, and the flow rate of the blood F₁ entering via the second inlet 301 may be increased. Thus, it can be seen that, in the present application, the blood flow rate pumped from the secondary pumping device 300 can be increased by providing the expanded diameter portion 323 between the proximal end portion 321 and the distal end portion 322 of the secondary cannula 320, so that most of the blood in the aorta 20 can be accelerated by the secondary pumping device 300, thus greatly improving a pumping efficiency of the secondary pumping device 300.

In addition, since the secondary cannula 320 has the expanded diameter portion 323, the secondary cannula 320 can also be gradually expanded from the distal end portion 322 to the expanded diameter portion 323, conforming to a streamlined flow of the blood, which facilitates the secondary cannula 320 to draw the blood into the interior of the secondary cannula 320 via the second inlet, allowing the drawn blood to flow rapidly to the expanded diameter portion 323; the secondary cannula 320 is tapered from the expanded diameter portion 323 to the proximal end portion 321, which facilitates the expanded diameter portion 323 to gradually squeezing the blood towards the proximal end portion 321, so that a blood pressure in the proximal end portion 321 is increased, thereby enabling rapid outward discharge through the second outlet at the proximal end portion 321. Thus, it can be seen that the shape of the secondary cannula 320 in the present application can also guide the inflow and the discharge of the blood, thus significantly reducing the blood flow resistance, effectively increasing a blood pumping volume, and improving blood pumping efficiency.

It is worth mentioning that a maximum diameter of the expanded diameter portion 323 is preferably less than an inner diameter of the aorta 20, so that the distance kept between the expanded diameter portion 323 and the inner wall surface of the aorta 20 is not zero, and the expanded diameter portion 323 is prevented from being attached to the aorta 20, thereby avoiding the aorta 20 from being dilated due to the secondary cannula 320 being located in the aorta 20 for a long time, and reducing the impact of the secondary pumping device 300 on a contraction performance of the aorta 20; in addition, the radial size of the secondary pumping device 300 can also be reduced, thus reducing the implantation difficulty.

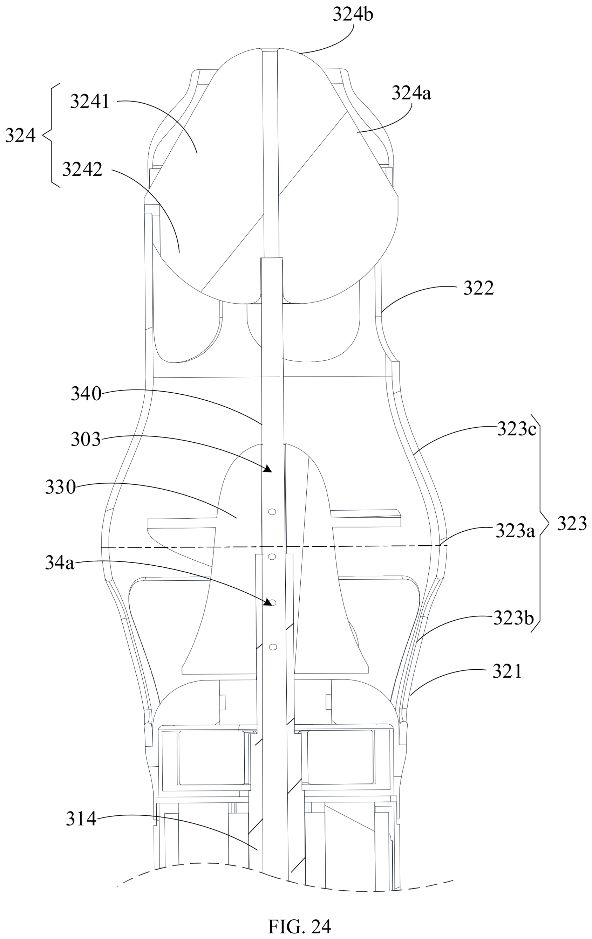

Referring to FIG. 24 to FIG. 27, in some embodiments, a circle at which the diameter of the expanded diameter portion 323 is maximum is taken as a reference circle 323a; a cannula wall of the expanded diameter portion 323 includes a first arc-shaped wall 323b and a second arc-shaped wall 323c; the first arc-shaped wall 323b has a diameter that gradually decreases from the reference circle 323a towards the proximal end portion 321; the second arc-shaped wall 323c has a diameter that gradually decreases from the reference circle 323a towards the distal end portion 322.

Specifically, the reference circle 323a is a virtual circle for dividing the first arc-shaped wall 323b and the second arc-shaped wall 323c. The expanded diameter portion 323 has and has only one reference circle 323a. The first arc-shaped wall 323b extends from the reference circle 323a to the proximal end portion 321, so that the expanded diameter portion 323 and the proximal end portion 321 are in a smooth transition; the second arc-shaped wall 323c extends from the reference circle 323a to the distal end portion 322, so that the expanded diameter portion 323 and the distal end portion 322 are in a smooth transition. This enables the cannula wall of the secondary cannula 320 to be smoother and more in line with the streamlined flow profile, so that the resistance encountered by the blood flowing in the secondary cannula 320 is smaller, blood collision is less likely to occur, a blood damage is reduced, and the blood pumping volume can be increased.

Further, the second outlet includes a plurality of outlet holes. The plurality of outlet holes are distributed and spaced apart in a circumferential direction of the secondary cannula 320; the outlet hole extends from the proximal end portion 321 of the secondary cannula 320 to the first arc-shaped wall 323b of the expanded diameter portion 323; a width of the outlet hole is gradually increased in an extending direction of the outlet hole.

When the blood flows into the first arc-shaped wall 323b of the expanded diameter portion 323 in the secondary cannula 320, at least part of the blood flows along the first arc-shaped wall 323b in wall-adherent motion. Since the diameter of the first arc-shaped wall 323b is gradually decreased from the reference circle 323a to the proximal end portion 321, the blood flowing along the first arc-shaped wall 323b in wall-adherent motion is gradually guided into the outlet hole by the first arc-shaped wall 323b, thus rapidly discharging the blood. In addition, since the width of the outlet hole is gradually increased in the extending direction thereof, that is, a maximum width end of the outlet hole is located on the first arc-shaped wall 323b, and a minimum width end of the outlet hole is located on the proximal end portion 321, so that the outlet hole can have a large outlet area, and the damage caused by contact between the blood and a periphery of the outlet hole can be reduced.

Referring to FIG. 24, FIG. 26 and FIG. 27, further, each of the outlet holes of the second outlet 302 has a first hole edge 302a, a second hole edge 302b and two third hole edges 302c; the first hole edge 302a is located on the first arc-shaped wall 323b, and the second hole edge 302b is located on the proximal end portion 321; an arc length of the first hole edge 302a extending in the circumferential direction of the secondary cannula 320 is greater than an arc length of the second hole edge 302b extending in the same direction; the two third hole edges 302c are linearly arranged and connected to the first hole edge and the second hole edge.

Specifically, since a circumferential length of the first arc-shaped wall 323b is large, the first arc-shaped wall 323b has a large outer peripheral surface area. The arc length of the first hole edge 302a is set to be greater than the arc length of the second hole edge 302b extending in the same direction, so that a distal end of the outlet hole has a large outlet area, which is beneficial to guiding and discharging the blood. The two third hole edges 302c are linearly arranged, so that the blood discharged from the distal end of the outlet hole can be guided to flow towards the outside of the proximal end portion 321, thereby increasing a component velocity of the blood flowing towards the second catheter 400, and improving the blood pumping efficiency.

In some embodiments, the secondary pumping device 300 further includes a secondary impeller 330. The secondary impeller 330 is arranged within the secondary cannula 320 and is rotatable relative to the secondary cannula 320; a proximal end of the secondary impeller 330 corresponds to the second outlet 302, and a distal end of the secondary impeller 330 extends into the expanded diameter portion 323 and is adjacent to the second inlet 301.

Specifically, a side surface of the proximal end of the secondary impeller 330 faces the second outlet 302; the distal end of the secondary impeller 330 extends through the expanded diameter portion 323 and is adjacent to the second outlet 302. That is, the distal end of the secondary impeller 330 is axially spaced apart from the second outlet 302 by a distance which is small. With such a design, the secondary impeller 330 may have a large axial length, the distal end of the secondary impeller 330 can drive the second inlet 301 to rapidly drawn the external blood, and the blood rotationally flows towards the proximal end of the secondary impeller 330 in the axial direction of the secondary impeller 330, and finally is centrifugally guided to the second outlet 302, so that working power of the secondary impeller 330 is greatly improved, thereby improving the blood pumping efficiency of the blood pump.

Referring to FIG. 27, in some embodiments, the proximal end portion 321 has a diameter D₆, the distal end portion 322 has a diameter D₇, and the expanded diameter portion 323 has a maximum diameter D₈; where 1.2 D₆≤D₈≤1.5 D₆, and D₇≤D₆. 1.2 D₆≤D₈≤1.5 D₆ means that D₈ is greater than or equal to 1.2 times D₆, and D₈ is less than or equal to 1.5 times D₆. Specifically, the reference circle 323a of the expanded diameter portion 323 has the maximum diameter D₈. If a difference between the maximum diameter D₈ of the expanded diameter portion 323 and the diameter D₆ of the proximal end portion 321 is too large, a transition between the expanded diameter portion 323 and the proximal end portion 321 is steep, so that collision with the blood pump is prone to occur to generate a vortex; if the difference between the maximum diameter D₈ of the expanded diameter portion 323 and the diameter D₆ of the proximal end portion 321 is too small, a flow guide effect of the expanded diameter portion 323 is not significant. Similarly, the same is true for a difference between the maximum diameter D₈ of the expanded diameter portion 323 and the diameter D₇ of the distal end portion 322. In view of this, 1.2 D₆≤D₈≤1.5 D₆ is preferable.

Referring to FIG. 5 and FIG. 29, for the primary pumping device 100, in some embodiments, the primary pumping device 100 includes a cannula assembly. The cannula assembly includes a primary cannula 120, an outlet tube 150 provided with the first outlet 102, and an inlet tube 140 provided with the first inlet 101. The primary cannula 120 connects the outlet tube 150 to the inlet tube 140. The outlet tube 150 is connected to a proximal end of the primary cannula 120, and the inlet tube 140 is connected to a distal end of the primary cannula 120. Optionally, the primary cannula 120 is an elastic tube; the secondary cannula 320 is a rigid tube.

Specifically, for the primary cannula 120, since the primary cannula 120 needs to be able to extend through the aorta 20 and the valve 21 thereof and be clamped by the valve 21, the primary cannula 120 needs to be configured as the elastic tube, so that the primary cannula 120 has better elasticity and can be adapted to a shape of the blood vessel and fully deformed, thereby facilitating the primary cannula 120 to enter the ventricle. Moreover, since the primary cannula 120 is clamped in the valve when the primary pumping device 100 extends through the valve of the aorta 20 to enter the ventricle, the elasticity of the primary cannula 120 can buffer a force between the valve and the primary cannula 120, reducing a reaction force on the valve, and avoiding the damage to the valve. Since the entire secondary pumping device 300 is located in the artery, and the secondary cannula 320 of the secondary pumping device 300 is not in contact with the valve 21, so that the secondary cannula 320 may be configured as the rigid tube. That is, the secondary cannula 320 may be made of a metal material, so that the secondary cannula 320 is not easily compressed and deformed, and smooth passage of the blood may be ensured.

In some embodiments, the primary pumping device 100 further includes the primary motor 110 and a primary impeller 130. The primary motor 110 is connected to the first catheter 200 and the outlet tube 150; the primary impeller 130 is arranged within the outlet duct 150 and connected to a rotating shaft of the primary motor 110. Optionally, power of the secondary motor 31 is less than or equal to that of the primary motor 110. With such a design, the primary pumping device 100 can be used to pump the blood to enable the bold to flow through the aortic arch, and the secondary motor 310 can be avoided from having excessive power, which is beneficial to reducing the volume of the secondary motor 310 and reducing the implantation difficulty.

In the blood pump 10 according to the above solution, by providing the primary pumping device 100, the first catheter 200, the secondary pumping device 300 and the second catheter 400 which are sequentially connected and in communication with each other, when the blood pump 10 is working, the blood in the ventricle sequentially flows through the first inlet 101 and the first outlet 102 of the primary pumping device 100, flows into the ascending part 22 of the aorta 20, and then flows through the second inlet 301 and the second outlet 302 of the secondary pumping device 300, and flows out to the descending part 23 of the aorta 20. Due to the presence of the secondary pumping device 300, the secondary pumping device 300 generates the negative pressure at the aortic arch, which causes the blood in the ascending part 22 of the aorta 20 to flow towards the descending part 23 of the aorta 20 in the accelerated manner, thereby increasing the blood flow rate. In addition, the diameter of the single pump (the primary pumping device 100 or secondary pumping device 300) can be reduced in the case of ensuring the adequate flow rate, thereby reducing the implantation difficulty.

In addition, since the second inlet 301 of the blood pump 10 according to the present application is arranged on the outer peripheral wall of the secondary pumping device 300, the second inlet 301 may have a large inlet area, thereby facilitating the blood in the aorta 20 to enter the secondary pumping device 300 via the second inlet 301, which can greatly reduce collision between the blood and the housing 311 of the secondary pumping device 300. In yet another aspect, since the proximal end of the first catheter 200 of the blood pump 10 is connected to the distal end of the secondary pumping device 300, if the second inlet 301 of the secondary pumping device 300 is arranged at the distal end of the secondary pumping device 300, blood flow at the second inlet 301 is prone to scour the junction between the first catheter 200 and the secondary pumping device 300, which is prone to cause the first catheter 200 to fall off. Therefore, in the blood pump 10 according to the present application, the second inlet 301 is arranged in the outer peripheral wall of the secondary pumping device 300, which not only can avoid interference with the first catheter 200, but also can reduce the scouring of the junction between the first catheter 200 and the secondary pumping device 300 by the blood flow at the second inlet 301, preventing the proximal end of the first catheter 200 from loosening.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the specification.

The above-described embodiments only illustrate several implementations of the present application, and the descriptions thereof are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the protection scope of the present application shall be subject to the appended claims.

## Claims

1. A blood pump, comprising a primary pumping device, a first catheter, a secondary pumping device and a second catheter which are connected in sequence;
wherein the primary pumping device is provided with a first inlet and a first outlet; the secondary pumping device is provided with a second inlet and a second outlet, and the second inlet is arranged in an outer peripheral wall of the secondary pumping device.

2. The blood pump according to claim 1, wherein the first catheter is internally provided with a first flushing tube, to supply flushing liquid to the primary pumping device, and the second catheter is internally provided with a second flushing tube, to supply the flushing liquid to the secondary pumping device; the secondary pumping device is internally provided with an intermediate channel, wherein the intermediate channel extends in an axial direction of the secondary pumping device and extends through the secondary pumping device, so as to be in communication with the first flushing tube and the second flushing tube.

3. The blood pump according to claim 2, wherein the secondary pumping device further comprises a secondary motor, a secondary cannula and a secondary impeller arranged within the secondary cannula; wherein the secondary motor is connected between the first catheter and the secondary cannula, the secondary motor comprises a rotating shaft, wherein an end of the rotating shaft is rotatably inserted into the secondary motor, another end of the rotating shaft extends through the secondary impeller to be rotatably connected to a distal end of the secondary cannula, and the rotating shaft is fixedly connected to the secondary impeller to drive the secondary impeller to rotate; the rotating shaft is configured as a hollow tube, and an inner cavity of the rotating shaft forms at least part of the intermediate channel.

4. The blood pump according to claim 2, wherein the secondary pumping device further comprises a secondary motor, a fixed tube, a secondary cannula and a secondary impeller arranged within the secondary cannula; wherein the secondary motor is connected between the first catheter and the secondary cannula; the fixed tube extends through the secondary impeller, the fixed tube has a first end and a second end, wherein the first end is fixed into the secondary motor, and the second end extends through the secondary impeller to be fixed to a distal end of the secondary cannula; the secondary impeller is rotatable about the fixed tube; the fixed tube is configured as a hollow tube, so that the fixed tube is internally hollow to form at least part of the intermediate channel.

5. The blood pump according to claim 4, wherein the secondary motor comprises: a housing; and a stator, a rotor and a rotating shaft that are mounted within the housing; wherein the rotating shaft is configured as a hollow tube and sleeved on an outer periphery of the fixed tube, a distal end of the rotating shaft extends out of the housing to be fixedly connected to the secondary impeller, and the rotating shaft is rotatable relative to the fixed tube; the rotor is fixedly connected to an outer peripheral surface of the rotating shaft; the stator is capable of generating a magnetic field that drives the rotor to rotate.

6. The blood pump according to claim 5, wherein the fixed tube has a first middle section extending through the rotating shaft, a first gap is formed between an outer peripheral surface of the first middle section and an inner peripheral surface of the rotating shaft, and two ends of the first gap are respectively in communication with the second flushing tube and a lumen of the secondary cannula.

7. The blood pump according to claim 6, wherein the secondary impeller is provided with a through hole extending through the secondary impeller in an axial direction of the secondary impeller, wherein the through hole has a large diameter section adjacent to the secondary motor and a small diameter section connected to the large diameter section; the distal end of the rotating shaft is inserted into and cooperates with the large diameter section of the through hole; the fixed tube further has a second middle section connected to the first middle section, wherein the second middle section is inserted into the small diameter section of the through hole, a second gap is formed between an outer peripheral surface of the second middle section and an inner peripheral surface of the small diameter section, and the second gap is in communication with the first gap and the lumen of the secondary cannula.

8. The blood pump according to claim 7, wherein the blood pump further has at least one of the following features:
a plurality of first draining holes extend through a side wall of the first middle section, and the first draining holes are in communication with the first gap;
a plurality of second draining holes extend through a side wall of the second middle section, and the second draining holes are in communication with the second gap;
one of the outer peripheral surface of the first middle section and the inner peripheral surface of the rotating shaft is provided with a flow guide structure, wherein the flow guide structure is located in the first gap, and the flow guide structure extends spirally in an axial direction of the rotating shaft;
a proximal bearing is arranged at a proximal end of the secondary motor, the proximal bearing is provided with a shaft hole, wherein the shaft hole has a first opening adjacent to the second flushing tube, a proximal end of the rotating shaft is inserted into the shaft hole, an end surface of the proximal end of the rotating shaft is axially spaced apart from the first opening to form a liquid inlet groove, wherein the liquid inlet groove is in communication with the first gap with the second flushing tube.

9. The blood pump according to claim 5, wherein at least one of an inner peripheral surface of the rotating shaft and an outer peripheral surface of the fixed tube is made of a metal material or a ceramic material; or, one of the inner peripheral surface of the rotating shaft and the outer peripheral surface of the fixed tube is provided with a magnetic ring, another of the inner peripheral surface of the rotating shaft and the outer peripheral surface of the fixed tube is provided with a magnet, and the magnetic ring and the magnet repel each other, so that the inner peripheral surface of the rotating shaft is suspended relative to the outer peripheral surface of the fixed tube.

10. The blood pump according to claim 2, wherein the primary pumping device comprises a primary motor and a cannula assembly connected to the primary motor, the primary motor being internally provided with a first flushing flow channel;
the secondary pumping device comprises a secondary motor and a secondary cannula connected to the secondary motor, and the intermediate channel extends from the secondary motor to a distal end of the secondary cannula to be in communication with the first flushing tube; the secondary motor is internally provided with a second flushing flow channel and a shunt flow channel, wherein the shunt channel is located at a proximal end of the secondary motor; the second flushing tube of the second catheter is connected to the shunt channel, so as to distribute flow through the shunt channel towards the second flushing flow channel and the intermediate channel.

11. The blood pump according to claim 10, wherein the secondary motor further comprises a fixing pin and a proximal bearing; the distal end of the secondary cannula is provided with a connecting end, the connecting end being fixedly connected to the first catheter; wherein the fixing pin is arranged at the proximal end of the secondary motor and fixedly connected to the second catheter; the proximal bearing is mounted to the fixing pin; a proximal end of the rotating shaft is inserted into and connected to the proximal bearing; a first end of the fixed tube extends through the proximal bearing and is fixedly connected to the fixing pin, and a second end of the fixed tube is fixedly connected to the connecting end.

12. The blood pump according to claim 11, wherein an end of the fixing pin is provided with a first positioning hole configured for insertion of the second flushing tube, and another end of the fixing pin is provided with a mounting hole configured for mounting of the proximal bearing; the shunt channel is arranged on the fixing pin, and the shunt channel comprises a liquid flow hole and a first shunt groove; wherein the liquid flow hole is arranged between the mounting hole and the first positioning hole of the fixing pin and in communication with the first positioning hole, the intermediate channel and the mounting hole; the first shunt groove is arranged in a side wall of the mounting hole and in communication the liquid flow hole and the second flushing flow channel.

13. The blood pump according to claim 12, wherein the fixing pin is further provided with a sunken groove and a second positioning hole; wherein the sunken groove is arranged in a bottom wall of the mounting hole and in communication with the first shunt groove, and the second positioning hole is arranged in a bottom wall of the sunken groove and configured for insertion of the fixed tube forming the intermediate channel; the shunt channel further comprises a second shunt groove and a third shunt groove; the second shunt groove is arranged in a side wall of the liquid flow hole; the third shunt groove is arranged in a side wall of the second positioning hole and sequentially in communication with the second shunt groove and the sunken groove.

14. The blood pump according to claim 1, wherein the secondary pumping device comprises a secondary cannula, the secondary cannula comprising a proximal end portion, a distal end portion and an expanded diameter portion; wherein the proximal end portion is adjacent to the second catheter and provided with the second outlet; the distal end portion is connected to the first catheter and provided with the second inlet; two ends of the expanded diameter portion are smoothly and transitionally connected to the proximal end portion and the distal end portion respectively.

15. The blood pump according to claim 14, wherein a circle at which a diameter of the expanded diameter portion is maximum is taken as a reference circle; a cannula wall of the expanded diameter portion comprises a first arc-shaped wall and a second arc-shaped wall; wherein a diameter of the first arc-shaped wall is gradually decreased from the reference circle to the proximal end portion; a diameter of the second arc-shaped wall is gradually decreased from the reference circle to the distal end portion.

16. The blood pump according to claim 15, wherein the second outlet comprises a plurality of outlet holes, the plurality of outlet holes being distributed and spaced apart in a circumferential direction of the secondary cannula; each of the outlet holes extends from the proximal end portion of the secondary cannula to the first arc-shaped wall, and a width of each of the outlet holes is gradually increased in the extending direction thereof;
or, the proximal end portion of the secondary cannula has a diameter D₆; the distal end portion of the secondary cannula has a diameter D₇, and the expanded diameter portion of the secondary cannula has a maximum diameter D₈; wherein 1.2 D₆≤D₈≤1.5 D₆, and D₇≤D₆.

17. The blood pump according to claim 1, wherein a distal end of the secondary pumping device is provided with a connecting end; a proximal end of the first catheter is provided with a first sleeving portion, wherein the first sleeving portion is sleeved on an outer peripheral surface of the connecting end and is fixedly connected to the connecting end; a distal end of the second catheter is provided with a second sleeving portion, and a proximal end of the secondary pumping device is provided with a fixing pin, wherein the second sleeving portion is sleeved on, connected and fixed to the fixing pin; the secondary pumping device is further internally provided with a fixed tube, wherein an end of the fixed tube is inserted and fixed to the fixing pin, and another end of the fixed tube is inserted and fixed to the connecting end.

18. The blood pump according to claim 17, wherein the first sleeving portion is configured to have a horn shape, and an inner diameter of the first sleeving portion is gradually increased in a direction from the first catheter to the secondary pumping device; the connecting end comprises a first variable-diameter peripheral surface, and the first variable-diameter peripheral surface is configured to conform to a shape of an inner peripheral surface of the first sleeving portion, so as to adapt to and be coupled to the first sleeving portion; or
the proximal end of the first catheter is further provided with a fixing cover, the fixing cover being sleeved on an outer periphery of the first sleeving portion, the fixing cover being provided with a reduced-diameter opening and an expanded-diameter opening, wherein a periphery of the expanded-diameter opening is fixedly connected to the connecting end; a periphery of the reduced-diameter opening is sleeved on the first catheter, and a diameter of the reduced-diameter opening is less than that of the first sleeving portion.

19. The blood pump according to claim 1, wherein the primary pumping device comprises a cannula assembly, the cannula assembly comprising a primary cannula, an outlet tube provided with the first outlet and an inlet tube provided with the first inlet, and the primary cannula being connected to the outlet tube and the inlet tube; the secondary pumping device comprises a secondary cannula, the secondary cannula being provided with the second inlet and the second outlet; wherein the primary cannula is an elastic tube, and the secondary cannula is a rigid tube; or, an outer peripheral wall of a distal end of the secondary cannula is provided with the second inlet, and the second inlet comprises a plurality of inlet holes, the plurality of inlet holes being distributed and spaced apart in a circumferential direction of the secondary cannula.

20. The blood pump according to claim 1, wherein the first catheter has a first outer diameter, the second catheter has a second outer diameter, and the fixed tube has a third outer diameter; wherein the third outer diameter is less than the first outer diameter, and the first outer diameter is less than the second outer diameter; or, the second outer diameter is less than twice the first outer diameter; or, the first catheter has a length ranging from 180 mm to300 mm to enable the secondary pumping device to be located in a descending part of an aorta.
